(19) Europäisches Patentamt
European Patent Office
Office européen des brevets

(11) Veröffentlichungsnummer: **0 090 362**
**A2**

# (12) EUROPÄISCHE PATENTANMELDUNG

(21) Anmeldenummer: **83102961.6**

(22) Anmeldetag: **24.03.83**

(51) Int. Cl.³: **A 61 K 37/02**, C 07 C 103/52, C 07 D 209/44, C 07 D 209/52

(30) Priorität: **30.03.82 DE 3211676**

(43) Veröffentlichungstag der Anmeldung: **05.10.83**
**Patentblatt 83/40**

(84) Benannte Vertragsstaaten: **AT BE CH DE FR GB IT LI LU NL SE**

(71) Anmelder: **HOECHST AKTIENGESELLSCHAFT,**
**Postfach 80 03 20, D-6230 Frankfurt am Main 80 (DE)**

(72) Erfinder: **Henning, Rainer, Dr., Völklinger Weg 56, D-6000 Frankfurt am Main 71 (DE)**
Erfinder: **Urbach, Hansjörg, Dr., Le Lavandoustrasse 41, D-6242 Kronberg/Taunus (DE)**
Erfinder: **Geiger, Rolf, Prof. Dr., Heinrich-Bleicher-Strasse 33, D-6000 Frankfurt am Main 50 (DE)**
Erfinder: **Teetz, Volker, Dr., An der Tann 20, D-6238 Hofheim am Taunus (DE)**
Erfinder: **Becker, Reinhard, Dr., Adelheidstrasse 101, D-6200 Wiesbaden (DE)**

(54) **Neue Derivate von Cycloalka(c)pyrrol-carbonsäuren, Verfahren zu ihrer Herstellung, diese enthaltende Mittel und deren Verwendung sowie neue Cycloalka(c)pyrrol-carbonsäuren als Zwischenstufen und Verfahren zu deren Herstellung.**

(57) Verbindungen der Formel I

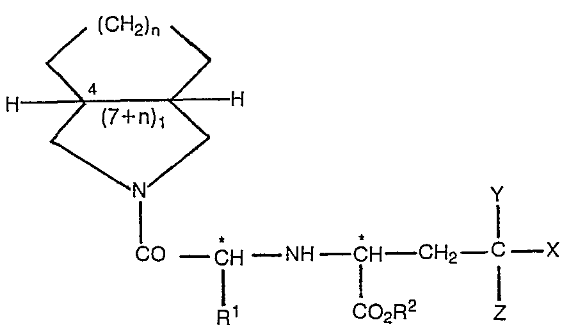

(I)

in der
n = 1, 2 oder 3,
$R^1$ = Wasserstoff, gegebenenfalls durch Amino, Acylamino oder Benzoylamino substituiertes Alkyl, Alkenyl, Cycloalkyl, Cycloalkenyl, Cycloalkyl-alkyl, Aryl oder teilhydriertes Aryl, das jeweils durch Alkyl, Alkoxy oder Halogen substituiert sein kann, Aryl-alkyl, das wie vorstehend definiert im Aryl-Rest substituiert sein kann, einen mono- bzw. bicyclischen Heterocyclen-Rest mit bis zu 10 Ringatomen, wovon 1 bis 2 Ringatome Schwefel- oder Sauerstoffatome und/oder wovon 1 bis 4 Ringatome Stickstoffatome darstellen oder eine Aminosäure-Seitenkette,
$R^2$ = Wasserstoff, Alkyl, Alkenyl oder Aryl-alkyl,
Y = Wasserstoff oder Hydroxy,
Z = Wasserstoff oder
Y und Z = zusammen Sauerstoff und
X = Alkyl, Alkenyl, Cycloalkyl, Aryl, das durch Alkyl, Alkoxy, Hydroxy, Halogen, Nitro, Amino, Alkylamino, Di-alkyl-amino oder Methylendioxy mono-, di- oder trisubstituiert sein kann, oder Indol-3-yl bedeuten, Verfahren zu ihrer Herstellung, diese enthaltende Mittel, ihre Verwendung sowie Zwischenstufen und Verfahren zu deren Herstellung.

EP 0 090 362 A2

HOECHST AKTIENGESELLSCHAFT HOE 82/F 065 Dr.WS/sch

Neue Derivate von Cycloalka/¯c_7pyrrol-carbonsäuren, Verfahren zu ihrer Herstellung, diese enthaltende Mittel und deren Verwendung sowie neue Cycloalka/¯c_7pyrrol-carbonsäuren als Zwischenstufen und Verfahren zu deren Herstellung

Die vorliegende Erfindung betrifft neue Derivate der bicyclischen Aminosäuren der Formel I

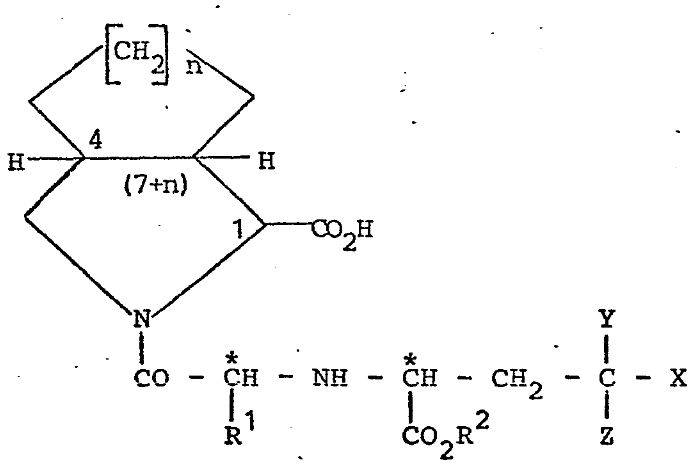

(I)

in der die Wasserstoffatome an den Brückenkopf-C-Atomen 4 und (7+n) zueinander cis- oder trans-konfiguriert sind, wobei die Carboxylgruppe an C-Atom 1 zum Wasserstoff-Atom an C-Atom 7+n cis- oder trans-ständig orientiert sein kann und worin

n = 1, 2 oder 3,

$R^1$ = Wasserstoff, $(C_1-C_6)$-Alkyl,
das gegebenenfalls durch Amino, $(C_1-C_4)$-Acylamino, vorzugsweise $(C_1-C_4)$-Alkanoylamino oder Benzoylamino substituiert sein kann, $(C_2-C_6)$-Alkenyl, $(C_5-C_9)$-Cycloalkyl, $(C_5-C_9)$-Cycloalkenyl, $(C_5-C_7)$-Cycloalkyl-$(C_1-C_4)$-alkyl, $(C_6-C_{10})$-Aryl oder teilhydriertes $(C_6-C_{10})$-Aryl, das jeweils durch $(C_1-C_2)$-Alkyl, $(C_1-C_2)$-Alkoxy oder Halogen substituiert sein kann, $(C_6-C_{10})$-Aryl-$(C_1-C_4)$-alkyl, dessen Arylrest, wie vorstehend definiert, substituiert sein kann, einen mono- bzw. bicyclischer Heterocyclenrest mit 5 bis 7 bzw. 8 bis 10 Ringatomen, wovon

1 bis 2 Ringatome Schwefel- oder Sauerstoffatome und/ oder wovon 1 bis 4 Ringatome Stickstoffatome darstellen, oder eine Seitenkette einer natürlich vorkommenden Aminosäure,

$R^2$ = Wasserstoff, $(C_1-C_6)$-Alkyl, $(C_2-C_6)$-Alkenyl oder $(C_6-C_{10})$-Aryl-$(C_1-C_4)$-alkyl,

Y = Wasserstoff oder Hydroxy,

Z = Wasserstoff oder

Y und Z = zusammen Sauerstoff,

X = $(C_1-C_6)$-Alkyl, $(C_2-C_6)$-Alkenyl, $(C_5-C_9)$-Cycloalkyl, $(C_6-C_{10})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkyl-amino oder Methylendioxy mono-, di- oder trisubstituiert sein kann, oder Indol-3-yl bedeuten,

sowie deren physiologisch unbedenkliche Salze.

Als Salze kommen insbesondere Alkali- und Erdalkalisalze, Salze mit physiologisch verträglichen Aminen und Salze mit anorganischen oder organischen Säuren wie z.B. HCl, HBr, $H_2SO_4$, Maleinsäure, Fumarsäure infrage.

Unter Aryl ist hier wie im folgenden vorzugsweise Phenyl oder Naphthyl zu verstehen. Alkyl kann geradkettig oder verzweigt sein.

Verbindungen der Formel I besitzen chirale C-Atome in den Positionen C-1, C-4, C-(7+n), sowie in den mit einem Stern markierten C-Atomen der Seitenkette. Sowohl die R- als auch die S-Konfigurationen an allen Zentren sind Gegenstand der Erfindung. Die Verbindungen der Formel I können daher als optische Isomere, als Diastereomere, als Racemate oder als Gemische derselben vorliegen. Bevorzugt sind die Verbindungen der Formel I, in denen das C-Atom 1 im bicyclischen Ringsystem, sowie die mit einem Stern markierten C-Atome der Seitenkette S-Konfiguration aufweisen.

Bevorzugte Verbindungen der Formel I sind solche, in denen

$R^1$ = Wasserstoff, $(C_1-C_3)$-Alkyl, $(C_2-C_3)$-Alkenyl, Benzyl 4-Amino-butyl, 4-Methoxybenzyl oder 4-Ethoxybenzyl,

$R^2$ = Wasserstoff, $(C_1-C_4)$-Alkyl oder Benzyl und

X = Phenyl, das durch $(C_1-C_2)$-Alkyl, $(C_1-C_2)$-Alkoxy, Hydroxy, Fluor, Chlor, Brom, Amino, $(C_1-C_4)$-Alkylamino, Di-$(C_1-C_4)$alkyl-amino, Nitro oder Methylendioxy, mono- oder disubstituiert oder im Falle von Methoxy trisubstituiert sein kann, bedeuten.

Insbesondere bevorzugt sind solche Verbindungen der Formel I, in denen n = 1 oder 2, $R^1$ = Methyl, 4-Methoxybenzyl oder 4-Ethoxybenzyl und X = Phenyl bedeuten, wobei solche, in denen $R^2$ = Wasserstoff oder Ethyl bedeutet, hervorzuheben sind.

Die Erfindung betrifft ferner Verfahren zur Herstellung der Verbindungen der Formel I. Eine Verfahrensvariante ist dadurch gekennzeichnet, daß man eine Verbindung der Formel II,

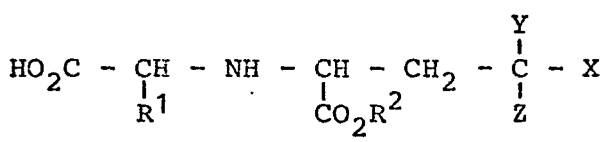

(II)

worin $R^1$, $R^2$, X, Y und Z die Bedeutungen wie in Formel I haben, mit einer Verbindung der Formel III,

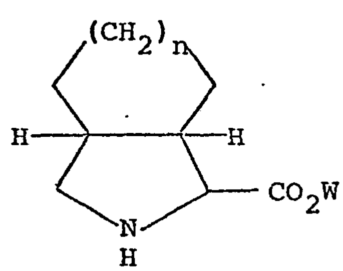

(III)

in der die Konfiguration des bicyclischen Ringsystems und des Substituenten an C-1 die gleiche ist wie in Formel I und worin

n = 1, 2 oder 3 und

W = einen hydrogenolytisch oder sauer abspaltbaren Rest, insbesondere einen Benzyl- oder einen tert.-Butyl-Rest bedeuten,

nach bekannten Amidbildungsmethoden der Peptidchemie umsetzt und anschließend durch katalytische Hydrierung oder Säure-Behandlung den Rest W und gegebenenfalls durch zusätzliche Säure- oder Basenbehandlung auch den Rest $R^2$ abspaltet, wobei jeweils die freien Carbonsäuren erhalten werden.

Weitere Syntheseverfahren zur Herstellung der Verbindungen der Formel I, in der Y und Z zusammen Sauerstoff bedeuten, bestehen darin, daß man eine Verbindung der Formel IV,

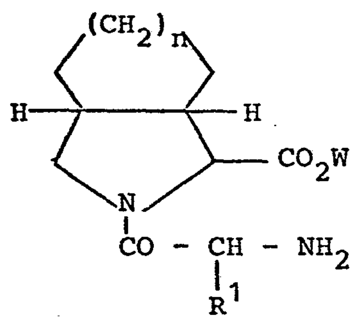

(IV)

in der die Konfiguration des bicyclischen Ringsystems und des Substituenten an C-1 die gleiche ist wie in Formel I und worin n und $R^1$ die Bedeutungen wie in Formel I besitzen und W die Bedeutung wie in Formel III hat, mit einer Verbindung der Formel V,

$$R^2O_2C - CH = CH - CO - X \qquad (V)$$

worin $R^2$ und X die Bedeutungen wie in Formel I besitzen in bekannter Weise in einer Michael-Reaktion (Organikum, 6. Auflage, S. 492, 1967) umsetzt und den Rest W und gegebenenfalls den Rest $R^2$ wie oben beschrieben abspaltet oder daß man eine Verbindung der obengenannten Formel IV mit einer Verbindung der allgemeinen Formel VI, worin $R^2$ die Bedeutung wie in Formel I hat, und mit einer Verbindung der allgemeinen Formel VII

$OHC-CO_2R^2$ (VI)

$X-CO-CH_3$ (VII)

worin X die Bedeutung wie in Formel I hat, in bekannter Weise in einer Mannich-Reaktion (s. Bull.Soc.Chim. France 1973, S. 625) umsetzt und anschließend den Rest W und gegebenenfalls den Rest $R^2$ wie oben beschrieben unter Bildung der freien Carboxygruppen abspaltet.

Ferner können Verbindungen der Formel I mit Y und Z = Wasserstoff auch in der Weise hergestellt werden, daß man eine Verbindung der obengenannten Formel IV gemäß der in J. Amer. Chem. Soc. 93, 2897 (1971) beschriebenen Verfahrensweise mit einer Verbindung der Formel VIII

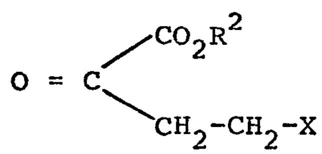

(VIII)

worin $R^2$ und X die Bedeutungen wie in Formel I besitzen, umsetzt, die erhaltenen Schiff-Basen reduziert und anschließend den Rest W und gegebenenfalls den Rest $R^2$ wie oben beschrieben, unter Bildung der freien Carboxygruppen abspaltet, oder daß man eine gemäß obigen Verfahrensweisen erhaltene Verbindung der Formel I, in der Y und Z zusammen Sauerstoff bedeuten, reduziert. Bevorzugt ist die katalytische Hydrierung. Die Reduktion der Schiff-Basen kann katalytisch, elektrolytisch oder mit Reduktionsmitteln wie beispielsweise komplexen Boranaten, vorzugsweise Natriumborhydrid oder Natriumcyanborhydrid erfolgen.

Verbindungen der Formel I mit Y = Hydroxy und Z = Wasserstoff können beispielsweise auch durch Reduktion einer gemäß obigen Verfahrensweisen erhaltenen Verbindung I mit Y und Z = zusammen Sauerstoff erhalten werden. Diese Reduktion kann katalytisch mit Wasserstoff oder mit einem anderen Reduktionsmittel wie beispielsweise komplexen Boranaten, vorzugsweise Natriumborhydrid erfolgen.

Gegenstand der Erfindung sind auch Verbindungen der Formel III',

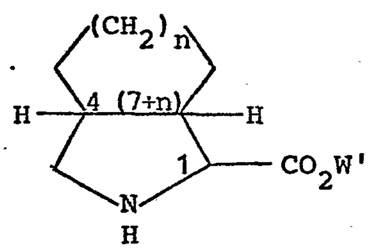

(III')

worin die C-Atome 1, 4 und (7+n) dieselben Konfigurationen wie in Formel III besitzen, n = 1, 2 oder 3 bedeuten und W' die Bedeutung von W in Formel III hat und zusätzlich Wasserstoff bedeutet. Diese Verbindungen dienen gemäß der Erfindung als Ausgangsstoffe bei der Synthese von Verbindungen der Formel I und können erfindungsgemäß nach folgenden Verfahrensweisen hergestellt werden.

Bei einer Synthesevariante geht man von einer Verbindung der Formel IX aus,

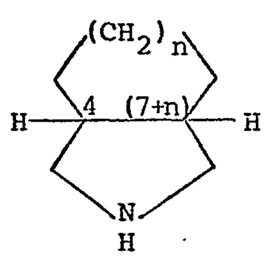

(IX)

in welcher die H-Atome an den C-Atomen 4 und (7+n) zueinander cis- oder trans-orientiert sind und n die Zahl 1, 2 oder 3 bedeutet.

Verbindungen der Formel IX mit n=1 sind aus R. Griot, Helv. Chim. Acta 42, 67 (1959), solche mit n=2 aus C.M. Rice et al., J. Org. Chem. 21, 1687 (1955) bekannt.

Diese Verbindungen der Formel IX werden in bekannter Weise acyliert, wobei ein aliphatischer oder aromatischer Acyl-Rest, vorzugsweise ein Acetyl- oder Benzoylrest an das Stickstoffatom gebunden wird, und die erhaltenen N-acylierten Verbindungen in einem aliphatischen Alkohol, bevorzugt einem Alkanol mit 1 bis 4 C-Atomen, insbesondere Methanol, in Gegenwart eines Leitsalzes vorzugsweise bei Temperaturen im Bereich von 0° bis +40°C unter Bildung einer Verbindung der Formel X, worin n = 1, 2, 3 und

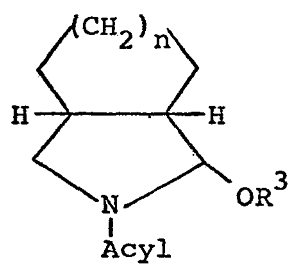

(X)

$R^3$ = $(C_1$-$C_4)$-Alkyl bedeuten, anodisch oxidiert (analog: Liebigs Ann. Chem. 1978, S. 1719).

Die erhaltene Verbindung der allgemeinen Formel X wird mit Trimethylsilylcyanid nach Tetrahedron Letters 1981, S. 141 in einem aprotischen organischen Lösemittel wie beispielsweise in einem Kohlenwasserstoff, Halogenkohlenwasserstoff, in Ether oder in THF bei Temperaturen im Bereich von -60°C bis +20°C, vorzugsweise -40°C bis $\pm$ 0°C in Gegenwart einer Lewis-Säure wie beispielsweise $ZnCl_2$, $SnCl_2$, $SnCl_4$, $TiCl_4$, $BF_3$-Etherat, vorzugsweise $BF_3$-Etherat, umgesetzt und die erhaltene Verbindung der Formel XI,

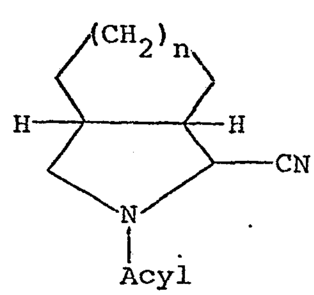

(XI)

worin die H-Atome an den C-Atomen 4 und 7+n zueinander cis- oder trans-ständig sind, wobei die CN-Gruppe sich in cis-Stellung zum H-Atom an C-Atom (7+n) befindet und worin n die oben genannte Bedeutung hat, nach Reinigung und Abtrennung von Nebenprodukten mittels Umkristallisation oder Säulenchromatographie durch Einwirkung von Säuren oder Basen in bekannter Weise zu einer Verbindung der Formel III mit W' = Wasserstoff hydrolysiert und letztere gegebenenfalls verestert. Bei der sauren Hydrolyse der Nitrilgruppe wird insbesondere HCl oder HBr als Säure verwendet. Die Veresterung wird hier wie im folgenden gemäß den in der Aminosäurenchemie üblichen Verfahrensweisen durchgeführt.

Verbindungen der allgemeinen Formel III' können auch hergestellt werden, indem man eine Verbindung der Formel XII,

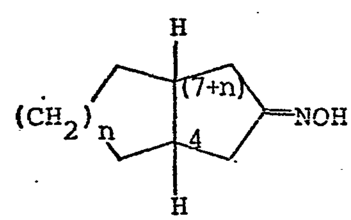

(XII)

in welcher die H-Atome an den C-Atomen 4 und (7+n) cis- oder trans-konfiguriert sind und n die oben genannte Bedeutung besitzt, in einer Beckmann-Umlagerung durch Umsatz mit einer Mineralsäure wie beispielsweise Schwefelsäure oder Polyphosphorsäure oder mit Benzolsulfonylchlorid oder p-Toluolsulfonylchlorid und einer Base wie Triethylamin in eine Verbindung der Formel XIII überführt, in welcher n die oben genannte Bedeutung besitzt,

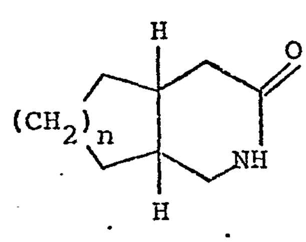

(XIII)

und diese zu einer Verbindung der Formel XIV,

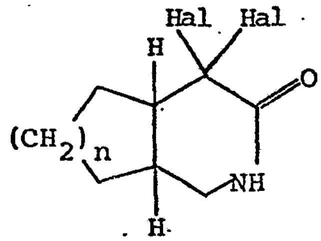

(XIV)

in welcher n die obengenannte Bedeutung besitzt und Hal ein Halogenatom, vorzugsweise Chlor oder Brom bedeutet, halogeniert. Als Halogenierungsmittel kommen beispielsweise anorganische Säurehalogenide wie $PCl_5$, $SO_2Cl_2$, $POCl_3$, $SOCl_2$, $PBr_3$ oder Halogene wie Brom in Betracht. Von Vorteil ist die Verwendung von $PCl_5$ oder $POCl_3$ in Kombination mit $SO_2Cl_2$. Als Zwischenstufe bildet sich zunächst ein Imidhalogenid, das mit den genannten Halogenierungsmitteln und anschließender Hydrolyse unter basischen Bedingungen, vorzugsweise mit wäßrigem Alkalicarbonat weiter zu einer Verbindung der Formel XIV reagiert.

Die Verbindungen der Formel XIV werden nachfolgend in einem polaren protischen Lösungsmittel wie beispielsweise einem Alkohol, vorzugsweise Ethanol, oder einer Carbonsäure wie beispielsweise Essigsäure unter Zusatz eines Säureacceptors wie beispielsweise Natriumacetat oder Triethylamin zu einer Verbindung der Formel XV

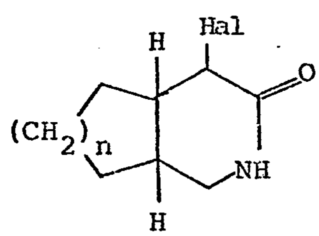

(XV)

in welcher n und Hal die oben genannten Bedeutungen besitzen, katalytisch reduziert. Als Katalysator kommen beispielsweise Raney-Nickel oder Palladium bzw. Platin auf Tierkohle in Betracht.

Verbindungen der Formel XV können auch direkt durch Halogenierung der Verbindungen der Formel XIII beim Einsatz geringerer Mengen der obengenannten Halogenierungsmittel hergestellt werden.

Verbindungen der Formel XV werden gemäß der bekannten Favorskii-Reaktion in Gegenwart einer Base zu einer Verbindung der Formel III' mit W' = Wasserstoff umgesetzt und diese gegebenenfalls verestert. Die obengenannte Favorskii-Reaktion wird in einem alkoholischen Lösungsmittel wie Methanol, Ethanol oder tert.-Butanol oder in Wasser oder in Gemischen derselben bei Temperaturen im Bereich von 20°C bis 140°C, vorzugsweise zwischen 60°C und 100°C durchgeführt. Als Basen werden zweckmäßig Alkali- oder Erdalkalihydroxide wie Natrium-, Kalium- oder Bariumhydroxid oder Alkalialkoholate wie beispielsweise Natriummethylat oder Kalium-tert.-butanolat eingesetzt.

Die Zwischenprodukte der Formel XIII können auch nach Can. J. Chem. 38, 557 (1960) aus ungesättigten Nitrilen der Formel XVI,

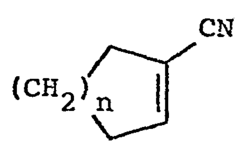

(XVI)

in welcher n die oben angegebene Bedeutung hat, durch Umsetzung mit Malonestern in einer Michael-Addition gefolgt von reduzierender Cyclisierung, Verseifung und Decarboxylierung hergestellt werden.

Die als Ausgangsstoffe zur Herstellung der Verbindungen der Formel I verwendeten Verbindungen der Formel II mit Y und Z = Wasserstoff, $R^1$ = Methyl und $R^2$ = Methyl oder Ethyl und X = Phenyl sind bekannt (europäische Patentanmeldung Nr. 37 231). Die Verbindungen der Formel II lassen sich nach verschiedenen Verfahrensweisen herstellen. Eine Synthesevariante geht von einem Keton der obengenannten Formel VII aus, das nach bekannten Verfahrensweisen in einer Mannich-Reaktion mit einer Verbindung der obengenannten Formel VI zusammen mit Aminosäureestern der Formel XVII

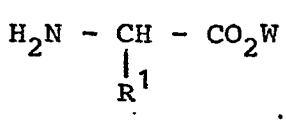

(XVII)

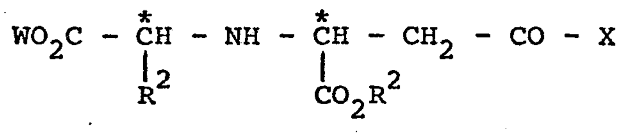

(XVIII)

worin $R^1$ und W die obengenannten Bedeutungen besitzen, zu einer Verbindung der Formel XVIII, worin $R^2$, $R^2$, X und W die obengenannten Bedeutungen besitzen, mit der Einschränkung, daß, falls W einen hydrogenolytisch abspaltbaren Rest, insbesondere Benzyl bedeutet, $R^2$ nicht die Bedeutung von W besitzen darf, umsetzt. Spaltet man den Rest W hydrogenolytisch mit Hilfe von beispielsweise Palladium ab, werden Verbindungen der Formel II mit Y und Z = Wasserstoff erhalten. Wird der Rest W mit Säuren wie beispielsweise Trifluoressigsäure oder Salzsäure in einem inerten organischen Lösungsmittel wie beispielsweise Dioxan abgespalten, erhält man Verbindungen der Formel II mit Y und Z zusammen = Sauerstoff.

Verbindungen der Formel XVIII sind auch durch Michael-Addition einer Verbindung der obengenannten Formel V mit einer Verbindung der obengenannten Formel XVII nach bekannten Verfahrensweisen zugänglich. Bevorzugt eignet sich dieses Verfahren zur Herstellung von solchen Verbindungen der Formel XVIII, in denen $R^1$ = Methyl, $R^2$ = Ethyl und X = Aryl bedeuten.

Die Verbindungen der Formel XVIII fallen als Diastereomerengemische an. Bevorzugte Diastereomeren der Formel XVIII sind solche, in denen die mit einem Stern markierten chiralen C-Atome jeweils S-Konfiguration aufweisen. Diese können durch Umkristallisieren oder durch Chromatographie,

beispielsweise an Kieselgel, abgetrennt werden. Bei der nachfolgenden Abspaltung des Restes W bleiben die Konfigurationen der chiralen C-Atome erhalten.

Die als Ausgangsstoffe zur Herstellung der Verbindungen der Formel I verwendeten Verbindungen der obengenannten Formel IV werden aus den Verbindungen der obengenannten Formel III durch Umsetzen mit einer N-geschützten 2-Aminocarbonsäure der Formel XIX

$$V - HN - \underset{\displaystyle R^1}{\underset{|}{CH}} - CO_2H \qquad (XIX)$$

worin V eine Schutzgruppe und $R^1$ die obengenannte Bedeutung besitzt, nach bekannten Verfahrensweisen erhalten. Als Schutzgruppe V, die nach beendeter Reaktion wieder abgespalten wird, kommen beispielsweise die Gruppen Benzyloxycarbonyl oder tert.-Butoxycarbonyl in Frage.

Die Ausgangsstoffe der Formel XII erhält man auf übliche Weise aus den bekannten Ketonen der Formel XX

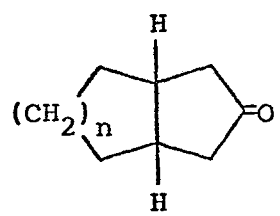

(XX)

wobei die H-Atome an den Brückenkopf-Kohlenstoffatomen cis- oder trans-ständig sein können und n die oben angegebene Bedeutung hat. Verbindungen der Formel XX mit n = 1 sind aus S. Dev, J. Indian Chem. Soc. 30, 815 (1953), solche mit n = 2 aus A. Kandiah, J. Chem. Soc. 922 (1931) und solche mit n = 3 aus A.M. Islam, R.A. Raphael, J. Chem. Soc. 315 (1955) bekannt.

Die Umsetzung einer Verbindung der Formel II mit einer Verbindung der Formel III zur Herstellung einer Verbindung der

Formel I erfolgt gemäß einer in der Peptidchemie bekannten Kondensationsreaktion, wobei als Kondensationsmittel beispielsweise Dicyclohexylcarbodiimid und 1-Hydroxy-benzotriazol zugesetzt werden. Bei der nachfolgenden hydrogenolytischen Abspaltung des Restes W wird als Katalysator bevorzugt Palladium verwendet, während bei der sauren Abspaltung des Restes W als Säuren bevorzugt Trifluoressigsäure oder Chlorwasserstoff eingesetzt werden.

In den oben beschriebenen Reaktionen zur Herstellung der Verbindungen der Formeln III', IV und I bleiben jeweils die Konfigurationen der Zwischenprodukte an den Brückenkopf-Atomen 4 und (7+n) erhalten. Die Ausgangsmaterialien der Formeln IX, XII und XIII entstehen bei der Synthese entweder als reine Diastereomere oder als Gemische der möglichen Diastereomeren, die gegebenenfalls durch Chromatographie oder Kristallisation getrennt werden können.

Die gemäß oben beschriebenen Verfahrensweisen erhaltenen Verbindungen der Formel III' fallen als racemische Gemische an und können als solche in die weiteren oben beschriebenen Synthesen eingesetzt werden. Sie können aber auch nach Auftrennung der Racemate mit üblichen Methoden beispielsweise über Salzbildung mit optisch aktiven Basen oder Säuren in die optischen Antipoden als reine Enantiomere eingesetzt werden.

Fallen die Verbindungen der Formel I als Racemate an, können auch diese nach den üblichen Methoden wie beispielsweise über Salzbildung mit optisch aktiven Basen oder Säuren in ihre Enantiomeren gespalten werden.

Die erfindungsgemäßen Verbindungen der Formel I liegen als innere Salze vor. Als amphotere Verbindungen können sie Salze mit Säuren oder Basen bilden. Diese Salze werden in üblicher Weise durch Umsetzen mit einem Äquivalent Säure bzw. Base hergestellt.

Die Verbindungen der Formel I und deren Salze besitzen lang andauernde, intensive blutdrucksenkende Wirkung. Sie sind starke Hemmer des Angiotensin-Converting-Enzyms (ACE-Hemmer). Sie können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1-100 mg, vorzugsweise bei 1-40 mg je Einzeldosis bei einem normalgewichtigen erwachsenen Patienten,*) Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

*)dies sind ca. 0,013-1,3 mg/kg/Tag, vorzugsweise 0,013-0,53 mg/kg/Tag.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche

Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol oder Glycerin, daneben auch Zuckerlösungen wie Glukose- oder Mannitlösungen, oder auch eine Mischung aus den verschiedenen genannten Lösungsmitteln.

Die außerordentlich starke Wirksamkeit der Verbindungen gemäß Formel I wird durch die pharmakologische Daten der nachfolgenden Tabelle belegt:

Intraduodenale (i.d.) bzw. intravenöse (i.v.) Gabe an der narkotisierten Ratte, 50 % Hemmung der durch 310 ng Angiotensin I ausgelösten Presserreaktion 30 min. nach Applikation in der Dosis ..... = $ED_{50}$:

Tabelle

Für alle aufgeführten Verbindungen gilt
X = Phenyl, $R^1$ = Methyl

| n | Y | Z | $R^2$ | Konfiguration des Bicyclus | $ED_{50}$ (mg/kg) | |
|---|---|---|---|---|---|---|
| 1 | H | H | $C_2H_5$ | 1S, 4S, 8S | 0,8 | (i.d.) |
| 1 | H | H | H | 1S, 4S, 8S | 0,2 | (i.v.) |
| 2 | H | H | $C_2H_5$ | 1S, 4S, 9S | 0,6 | (i.d.) |
| 3 | H | H | $C_2H_5$ | 1S, 4S, 10S | 1,8 | (i.d.) |
| 1 | H | H | $C_2H_5$ | 1S, 4R, 8S | 1,0 | (i.d.) |
| 2 | H | H | H | 1S, 4R, 9S | 0,3 | (i.v.) |
| 2 | H | H | $C_2H_5$ | 1S, 4R, 9S | 1,2 | (i.d.) |
| 3 | H | H | $C_2H_5$ | 1S, 4R, 10S | 2,1 | (i.d.) |
| 3 | H | H | H | 1S, 4S, 10S | 0,5 | (i.v.) |
| 3 | H | H | H | 1S, 4R, 10S | 0,6 | (i.v.) |
| 1 | - O - | | $C_2H_5$ | 1S, 4S, 8S | 1,1 | (i.d.) |
| 2 | - O - | | $C_2H_5$ | 1S, 4S, 9S | 1,4 | (i.d.) |

Die Symbole n, X, Y, Z, $R^1$ und $R^2$ beziehen sich auf die Verbindungen der Formel I.

Die nachfolgenden Beispiele dienen zur Erläuterung der Erfindung, ohne diese auf die stellvertretend genannten Verbindungen zu beschränken.

Beispiel 1

(±)-(1S,4S,8S)-Octahydrocyclopenta /c_7pyrrol-1-carbonsäure

a) N-Acetyl-cis-octahydrocyclopenta/c_7pyrrol

Zu einer Lösung von 3 g Octahydrocyclopenta/c_7pyrrol und 4,2 ml Triethylamin in 30 ml trockenem Tetrahydrofuran werden bei 0°C 2,1 ml Acetylchlorid getropft. Nach 15 min Rühren bei 0°C wird mit 150 ml Wasser verdünnt, mit 1 N Salzsäure sauer gestellt und mit Methylenchlorid extrahiert. Die organische Phase wird getrocknet und eingeengt. Das Rohprodukt wird im Hochvakuum destilliert.

Ausbeute: 2,4 g; Kp. 71°C/0,2 mm

b) N-Acetyl-2-methoxy-cis-octahydrocyclopenta/c_7pyrrol

2,4 g N-Acetyl-cis-octahydrocyclopenta/c_7pyrrol werden in Methanol unter Zusatz von Tetramethylammoniumtetrafluroborat gemäß den Angaben in Liebigs Ann.Chem. 1978, Seite 1719 anodisch oxidiert. Das Lösungsmittel wird entfernt, der Rückstand in Ether aufgenommen, filtriert und das Filtrat eingeengt.

$^1$H-NMR-Daten: 3,33 + 3,27 (2s,$OCH_3$)
2,12 + 2,06 (2s,$COCH_3$)

c) (±)-(1S,4S,8S)-N-Acetyl-octahydrocyclopenta/c_7-pyrrol-1-carbonsäurenitril

Zu einer Lösung von 2.4 g N-Acetyl-2-methoxy-cis-octahydrocyclopenta/c_7pyrrol in 15 ml Methylenchlorid werden bei -40°C 1,31 g Trimethylsilylcyanid in 3 ml Methylenchlorid gegeben. Anschließend tropft man 1,88 g Bortrifluorid-etherat zu, wobei die Temperatur auf

-30°C ansteigt. Nach 2 Stunden bei -20°C und 2 Stunden bei 0°C versetzt man mit Wasser und extrahiert dreimal mit Methylenchlorid. Die organische Phase wird mit Natrium-bicarbonatlösung und Wasser sowie gesättigter Kochsalzlösung gewaschen, getrocknet und eingeengt. Man erhält 2,2 g der Titelverbindung als farbloses Öl.

d) (±)-(1S,4S,8S)-octahydrocyclopenta/c_7pyrrol-1-carbonsäure

2,2 g (±)-(1S,4S,8S)-N-Acetyl-octahydrocyclopenta/c_7-1-carbonsäure-nitril werden in 8 ml konz. Bromwasserstoff 2 Std. zum Sieden erhitzt. Nach Abdestillieren des Bromwasserstoffs wird der Rückstand mit wenig Aceton verrührt und abgesaugt. Eine wäßrige Lösung des Produktes wird mit einem schwach basischen Ionenaustauscher auf einen pH-Wert von 6,0 gestellt. Nach Filtration wird die Lösung eingedampft und der Rückstand über Kieselgel mit einem Gemisch von Methylenchlorid, Methanol, Eisessig und Wasser im Verhältnis 20 : 10 : 0,5 : 0,5 filtriert. Das Eluat wird eingeengt und der Rückstand mit Diisopropyläther verrieben.

Ausbeute: 1,6 g, Fp. 190 - 195°C

Verfährt man analog den unter Beispiel 1 beschriebenen Verfahrensweisen, so erhält man die unter Beispiel 2 und Beispiel 3 im folgenden genannten Verbindungen:

Beispiel 2

(±)-(1S,4S,8S)-Octahydro-2H-cyclohexa/c_7-pyrrol-1-carbonsäure

a) N-Acetyl-cis-octahydro-2H-isoindol

| | | |
|---|---|---|
| $^1$H-NMR-Daten ($CDCl_3$): | 1,0 bis 2,3 | (m,1oH) |
| | 2,0 | (2s, 3H) |
| | 3,3 bis 4,2 | (m, 4H) |

b) N-Acetyl-1-methoxy-cis-octahydro-2H-isoindol

| | | |
|---|---|---|
| $^1$H-NMR-Daten ($CDCl_3$): | 0,9 - 2,6 | (m, 1OH) |
| | 2,1 | (s, 3H) |
| | 3,3 | (s, 3H) |
| | 3,5 - 4,0 | (m, 2H) |
| | 4,7 - 5,5 | (m, 1H) |

c) N-Acetyl-1-cyano-cis-octahydro-2H-isoindol

| | | |
|---|---|---|
| $^1$H-NMR-Daten ($CDCl_3$): | 1,0 - 3,0 | (m, 1OH) |
| | 2,0 | (2S, 3H) |
| | 3,3 - 3,9 | (m, 2H) |
| | 4,4 - 4,7 | (m, 1H) |

d) (±)-(1S,4S,9S)-Octahydro-2H-cyclohexa/c_7-pyrrol-1-carbonsäure

| | | |
|---|---|---|
| $^1$H-NMR-Daten ($D_2O$): | 1,0 - 3,5 | (m, 12H) |
| | 3,8 - 4,3 | (m, 1H) |

Beispiel 3

(±)-(1S,4S,10S)-Decahydro-cyclohepta /c_7pyrrol-1-carbonsäure

a) N-Acetyl-cis-decahydrocyclohepta/c 7pyrrol

| | | |
|---|---|---|
| $^1$H-NMR-Daten ($CDCl_3$): | 1,0 - 2,4 | (m, 12H) |
| | 2,0 | (2s, 3H) |
| | 3,3 - 4,2 | (m, 4H) |

b) N-Acetyl-1-methoxy-cis-decahydrocyclohepta/c_7pyrrol

$^1$H-NMR-Daten (CDCl$_3$):

| | | |
|---|---|---|
| 0,9 | - 2,5 | (m, 12H) |
| 2,1 | | (s, 3H) |
| 3,3 | | (s, 3H) |
| 3,5 | - 4,0 | (m, 2H) |
| 4,7 | - 5,5 | (m, 1H) |

c) N-Acetyl-1-cyano-cis-decahydrocyclohepta/c_7pyrrol

$^1$H-NMR-Daten (CDCl$_3$):

| | | |
|---|---|---|
| 1,0 | - 3,0 | (m, 12H) |
| 2,0 | | (2s, 3H) |
| 3,3 | - 3,9 | (m, 2H) |
| 4,4 | - 4,7 | (m, 1H) |

d) (±)-(1S,4S,10S)-Decahydrocyclohepta/c_7pyrrol-1-carbonsäure

$^1$H-NMR-Daten (D$_2$O):

| | | |
|---|---|---|
| 0,8 | - 3,4 | (m, 14H) |
| 3,8 | - 4,3 | (m, 1H) |

Beispiel 4

(±)-(1S,4S,9S)-Octahydro-2H-cyclohexa-/c_7pyrrol-1-carbonsäure

a) Cis-Octahydro-2H-isochinolin-3-on

Zu einer Mischung aus 33,6 g (0,22 Mol)cis-Hexahydro-indan-2-on-oxim und 300 ml 5 N Natronlauge werden 41 g (0,24 Mol) Benzolsulfonylchlorid gegeben, wobei die Temperatur unter 50°C gehalten wird. Eine Stunde nach

Beendigung der Zugabe wird mit Methylenchlorid extrahiert, getrocknet und eingeengt, der Rückstand wird bei 100-110 °C/20 mm sublimiert,
Ausbeute: 20 g, Fp. 149 - 151 °C

b) 4,4-Dichlor-cis-octahydro-2H-isochinolin-3-on

Eine Lösung von 23 g cis-Octahydro-2H-isochinolin-3-on in 350 ml wasserfreiem Chloroform wird mit 28,8 g Phosphorpentachlorid bei Raumtemperatur versetzt. Hierzu werden 43,1 g Sulfurylchlorid in 45 ml wasserfreiem Chloroform innerhalb von 30 Min. bei 20 bis 30 °C tropfenweise gegeben und das Reaktionsgemisch 5 Stunden bei Siedetemperatur gerührt. Nach Stehenlassen über Nacht wird das Gemisch mit auf 0 °C gekühltem wäßrigem Kaliumcarbonat neutral gestellt. Die wäßrige Phase wird 2 mal mit Methylenchlorid extrahiert. Die vereinigten organischen Phasen werden über Natriumsulfat getrocknet und eingeengt. Der Rückstand wird aus Äthanol unter Zusatz von Aktivkohle umkristallisiert. Man erhält 32 g blaßgelbe Kristalle.

c) 4-Chlor-cis-octahydro-2H-isochinolin-3-on

15,9 g 4,4-Dichlor-cis-octahydro-2H-isochinolin-3-on werden in einem Liter Ethanol unter Zusatz von 10 ml Triethylamin und Raney-Nickel bei 20 bis 25 °C unter Normaldruck bis zur Aufnahme von einem Mol-Äquivalent Wasserstoff hydriert. Nach Filtration wird die Lösung eingedampft, der Rückstand in Essigester aufgenommen, die Lösung zweimal mit Wasser extrahiert und über Natriumsulfat getrocknet. Nach Entfernen des Lösungsmittels wird das Produkt mit Diisopropyläther verrieben und abgesaugt. Es werden farblose Kristalle der Titelverbindung erhalten.

d) (±)-(1S,4S,9S)-Octahydro-2H-cyclohexa/c_7pyrrol-1-carbonsäure

3,75 g 4-Chlor-cis-octahydro-2H-isochinolin-3-on werden in eine siedende Lösung von 6,63 g Bariumhydroxidoctahydrat in 120 ml Wasser gegeben. Nach 3,5 stündigem Erhitzen unter Rückfluß wird das Reaktionsgemisch mit 0,9 ml konz. Schwefelsäure versetzt, eine weitere Stunde zum Sieden erhitzt und zur Koagulation anschließend über Nacht stehen gelassen.

Der ausgefallene Niederschlag wird abgesaugt und das auf einen pH-Wert von 6,5 gestellte Filtrat zur Trockene eingedampft. Der Rückstand wird mit siedendem Ethanol extrahiert, eingeengt und zur Kristallisation gebracht. Ausbeute: 3,1 g (identisch mit der in Beispiel 2d) beschriebenen Verbindung).

Beispiel 5

(±)-(1S,4R,9S)-Octahydro-2H-cyclohexa/c_7pyrrol-1-carbonsäure

a) trans-Octahydro-2H-isochinolin-3-on

Analog Beispiel 4a) werden 14,1 g trans-Hexahydroindan-2-on-oxim mit 17,2 g Benzolsulfonylchlorid zu 8 g der Titelverbindung umgesetzt,
Fp: 165 - 167°C

b) 4,4-Dichlor-trans-octahydro-2H-isochinolin-3-on

Analog Beispiel 4b) werden 7,1 g trans-Octahydro-2H-isochinolin-3-on durch Umsetzung mit 8,9 g Phosphorpentachlorid und 14 ml Sulfurylchlorid zu 11 g der Titelverbindung umgewandelt: blaßgelbe Kristalle, F. 133 - 137°C.

c) 4-Chlor-trans-octahydro-2H-isochinolin-3-on

Analog Beispiel 4c) werden 8 g 4,4-Dichlor-trans-octahydro-2H-isochinolin-3-on durch Hydrierung in 5 g der Titelverbindung überführt.

d) (±)-(1S,4R,9S)-Octahydro-2H-cyclohexa/¯c_7pyrrol-1 carbonsäure

Analog Beispiel 4d) erhält man aus 4,2 g 4-Chlor-trans-octahydro-2H-isochinolin-3-on 3,4 g der Titelverbindung als farbloses, amorphes Pulver.

$^1$H-NMR-Daten ($D_2O$): 0,8 - 3,4 (m,12H)
3,6 - 4,3 (m, 1H)

Die nachfolgenden Aminosäuren lassen sich in analoger Weise zu der in Beispiel 4a) bis d) beschriebenen Verfahrensweise herstellen.

Beispiel 6

(±)-(1S,4S,8S)-Octahydro-cyclopenta/¯c_7pyrrol-1-carbonsäure

Farblose Kristalle, Fp. 190 - 195°C (identisch mit der in Beispiel 1d) beschriebenen Verbindung).

Beispiel 7

(±)-(1S,4R,8S)-Octahydro-cyclopenta/¯c_7pyrrol-1-carbonsäure

Farbloses amorphes Pulver

$^1$H-NMR-Daten ($D_2O$): 0,9 - 3,2 (m, 10H)
3,5 - 4,4 (m, 1H)

Beispiel 8

(±)-(1S,4S,10S)-Decahydro-cyclohepta/c_7pyrrol-1-carbonsäure

Farbloses amorphes Pulver.

$^1$H-NMR-Daten ($D_2O$): 1,0 - 3,4 (m, 14H)
3,4 - 4,4 (m, 1H)

Beispiel 9

(±)-(1S,4R,10S)-Decahydro-cyclohepta/c_7pyrrol-1-carbonsäure

Farbloses amorphes Pulver

$^1$HNMR-Daten ($D_2O$): 1,0 - 3,6 (m, 14H)
3,7 - 4,1 (m, 1H)

Beispiel 10

(±)-(1S,4S,8S)-Octahydrocyclopenta/c_7pyrrol-1-carbonsäure-benzylester-hydrochlorid

Zu einer bei -40°C hergestellten Lösung von 0,7 ml Thionylchlorid in 7 ml Benzylalkohol werden bei -5°C 0,7 g (±)-(1S,4S,8S)-Octahydro-cyclopenta/c_7pyrrol-1-carbonsäure gegeben. Nach 20 Stunden Reaktionszeit bei Raumtemperatur wird der Benzylalkohol entfernt und der Rückstand mit Diisopropylether verrieben. Man erhält 0,84 g der Titelverbindung als farblose Kristalle vom Fp. 120 - 122°C

| | | | |
|---|---|---|---|
| $^1$H-NMR-Daten (DMSO-$d_6$): | 1,1 | - 2,3 | (m, 8H) |
| | 3,2 | - 3,7 | (m, 2H) |
| | 3,8 | - 4,2 | (m, 1H) |
| | 5,1 | | (s, 2H) |
| | 7,2 | | (s, 5H) |

Analog zu der in Beispiel 10 beschriebenen Verfahrensweise lassen sich die nachfolgenden Benzylester-derivate der Beispiele 11 bis 15 herstellen:

## Beispiel 11

(±)-(1S,4S,9S)-Octahydrocyclohexa/¯c¯7pyrrol-1-carbonsäure-benzylester-hydrochlorid

| | | | |
|---|---|---|---|
| $^1$H-NMR-Daten (DMSO-$d_6$): | 1,1 | - 2,4 | (m, 10H) |
| | 3,2 | - 3,7 | (m, 2H) |
| | 3,8 | - 4,3 | (m, 1H) |
| | 5,1 | | (s, 2H) |
| | 7,2 | | (s, 5H) |

## Beispiel 12

(±)-(1S,4S,10S)-Decahydrocyclohepta/¯c¯7-1-carbonsäure-benzylester-hydrochlorid

| | | | |
|---|---|---|---|
| $^1$H-NMR-Daten (DMSO-$d_6$): | 0,9 | - 2,4 | (m, 12H) |
| | 3,2 | - 3,7 | (m, 2H) |
| | 3,8 | - 4,4 | (m, 1H) |
| | 5,1 | | (s, 2H) |
| | 7,2 | | (s, 5H) |

Beispiel 13

(±)-(1S,4R,8S)-Octahydrocyclopenta/c_7pyrrol-1-carbonsäure-benzylester-hydrochlorid

$^1$H-NMR-Daten (DMSO-$d_6$): 1,0 - 2,2 (m, 8H)
3,0 - 3,7 (m, 2H)
3,8 - 4,4 (m, 1H)
5,2 (s, 2H)
7,2 (s, 5H)

Beispiel 14

(±)-(1S,4R,9S)-Octahydrocyclohexa/c_7pyrrol-1-carbonsäure-benzylester-hydrochlorid

$^1$H-NMR-Daten (DMSO-$d_6$): 1,1 - 2,2 (m, 10H)
3,0 - 3,6 (m, 2H)
3,8 - 4,6 (m, 1H)
5,1 (s, 2H)
7,2 (s, 5H)

Beispiel 15

(±)-(1S, 4R,10S)-Decahydrocyclohepta/c_7pyrrol-1-carbonsäure-benzylester-hydrochlorid

$^1$H-NMR-Daten (DMSO-$d_6$): 1,0 - 2,4 (m, 12H)
3,0 - 3,6 (m, 2H)
3,7 - 4,4 (m, 1H)
5,1 (s, 2H)
7,2 (s, 5H)

Beispiel 16

(±)-(1S,4S,8S)-Octahydrocyclopenta/c_7pyrrol-1-carbonsäure-tert.-butylester-hydrochlorid

Zu einer auf -10°C gekühlten Lösung von 10 g (±)-(1S,4S,8S)-Octahydrocyclopenta/c_7pyrrol-1-carbonsäure in 100 ml Dioxan werden 10 ml konz. Schwefelsäure und 50 g Isobutylen gegeben. Das Reaktionsgemisch wird im Autoklaven langsam auf 20 bis 25°C erwärmt und 20 Stunden bei dieser Temperatur gerührt.

Man gibt das Gemisch in eiskaltes 50%iges wäßriges Natriumhydroxid und extrahiert mit Methylenchlorid. Die vereinigten organischen Phasen werden mit Wasser gewaschen, mit Natriumsulfat getrocknet und eingeengt. Der Rückstand wird in Ether aufgenommen und mittels etherischem Chlorwasserstoff auf einen pH-Wert von 2,0 bis 3,0 gestellt. Man engt zur Trockene ein und verreibt das Produkt mit Diisopropylether. Nach Absaugen werden 7,3 g der Titelverbindung erhalten.

| | | | | |
|---|---|---|---|---|
| $^1$H-NMR-Daten (DMSO-$d_6$): | 1,0 | - | 2,4 | (m, 8H) |
| | 1,3 | | | (s, 9H) |
| | 3,2 | - | 3,6 | (m, 2H) |
| | 3,8 | - | 4,4 | (m, 1H) |

Die tert.-Butylester der nachfolgenden Beispiele 17 bis 19 können analog hergestellt werden:

Beispiel 17

(±)-(1S,4S,9S)-Octahydrocyclohexa/c_7pyrrol-1-carbonsäure-tert.-butylester-hydrochlorid

| | | | | |
|---|---|---|---|---|
| $^1$H-NMR-Daten (DMSO-$d_6$): | 1,1 | - | 2,7 | (m, 10H) |
| | 1,3 | | | (s, 9H) |

| | | | |
|---|---|---|---|
| 3,2 | - | 3,7 | (m, 2H) |
| 3,8 | - | 4,5 | (m, 1H) |

**Beispiel 18**

**(±)-(1S,4R,8S)-Octahydrocyclopenta/c_7pyrrol-1-carbonsäure-tert.-butylester-hydrochlorid**

$^1$H-NMR-Daten (DMSO-$d_6$):

| | | | |
|---|---|---|---|
| 1,1 | - | 2,8 | (m, 8H) |
| 1,3 | | | (s, 9H) |
| 3,2 | - | 3,6 | (m, 2H) |
| 3,7 | - | 4,5 | (m, 1H) |

**Beispiel 19**

**(±)-(1S,4R,9S)-Octahydrocyclohexa/c_7pyrrol-1-carbonsäure-tert.-butylester-hydrochlorid**

$^1$H-NMR-Daten (DMSO-$d_6$)

| | | | |
|---|---|---|---|
| 1,1 | - | 3,0 | (m, 10H) |
| 1,3 | | | (s, 9H) |
| 3,1 | - | 3,5 | (m, 2H) |
| 3,7 | - | 4,5 | (m, 1H) |

**Beispiel 20**

**N-(1S-Carbethoxy-3-phenyl-propyl)-S-alanyl-1S,4S,8S-octahydrocyclopenta/c_7pyrrol-1-carbonsäurebenzylester (=Diastereomer A 20) und N-(1S-Carbethoxy-3-phenyl-propyl)-S-alanyl-1R,4R,8R-octahydrocyclopenta/c_7pyrrol-1-carbonsäurebenzylester (=Diastereomer B 20)**

0,82 g N-(1S-Carbethoxy-3-phenyl-propyl)-S-alanin, 0,39 g

1-Hydroxy-benztriazol, 0,82 g (±)-(1S,4S,8S)-Octahydro-cyclopenta/c_7pyrrol-1-carbonsäurebenzylester-hydrochlorid, 0,5 ml N-Ethylmorpholin und 0,59 g Dicyclohexylcarbodiimid werden bei 20°C in 4 ml Dimethylformamid gegeben. Man rührt 3 Stunden, verdünnt mit 50 ml Essigester, extrahiert zweimal mit Wasser, trocknet über Natriumsulfat und engt ein.

Es wird ein Gemisch der obengenannten Diastereomeren A 20 und B 20 erhalten, welcher über Kieselgel mit einem Gemisch von Cyclohexan/Essigester (2:1) getrennt wird.

$R_f$-Wert für Diastereomer A 20: 0,22
$R_f$-Wert für Diastereomer B 20: 0,20

Die Verbindungen der nachstehenden Beispiele 21 bis 29 werden in analoger Verfahrensweise, wie in Beispiel 20 beschrieben, hergestellt:

## Beispiel 21

N-(1-S-Carbethoxy-3-phenyl-propyl)-S-alanyl-1S,4R,8S-octahydro-cyclopenta/c_7pyrrol-1-carbonsäure-benzylester (=Diastereomer A 21)

| | | |
|---|---|---|
| $^1$H-NMR-Daten ($CDCl_3$): | 1,25 | (d+t 6H) |
| | 1,3 - 2,4 | (m, 10H) |
| | 2,3 - 3,4 | (m, 6H) |
| | 4,1 | (g, 2H) |
| | 4,6 | (d, 1H) |
| | 5,2 | (s, 2H) |
| | 7,2 | (s, 5H) |
| | 7,4 | (s, 5H) |

Beispiel 22

N-(1-S-Carbethoxy-3-phenyl-propyl)-S-alanyl-1S,4S,9S-octahydrocyclohexa/c_7pyrrol-1-carbonsäure-benzylester (=Diastereomer A 22)

| | | |
|---|---|---|
| $^1$H-NMR-Daten ($CDCl_3$): | 1,1 | (d, 3H) |
| | 1,3 | (t, 3H) |
| | 1,4 - 2,5 | (m, 12H) |
| | 2,3 - 3,4 | (m, 6H) |
| | 4,2 | (q, 2H) |
| | 4,5 | (d, 1H) |
| | 5,1 | (s, 2H) |
| | 7,2 | (s, 5H) |
| | 7,35 | (s, 5H) |

Beispiel 23

N-(1-S-Carbethoxy-3-phenyl-propyl)-S-alanyl-1S,4R,9S-octahydrocyclohexa/c_7pyrrol-1-carbonsäure-benzylester (= Diastereomer A 23)

| | | |
|---|---|---|
| $^1$H-NMR-Daten ($CDCl_3$): | 1,2 | (d+t, 6H) |
| | 1,3 - 2,5 | (m, 12H) |
| | 2,3 - 3,4 | (m, 6H) |
| | 4,1 | (q, 2H) |
| | 4,5 | (d, 1H) |
| | 5,2 | (s, 2H) |
| | 7,2 | (s, 5H) |
| | 7,35 | (s, 5H) |

Beispiel 24

N-(1-S-Carbethoxy-3-phenyl-propyl)-S-alanyl-1S,4S,10S-decahydrocyclohepta/c 7pyrrol-1-carbonsäurebenzylester (= Diastereomer A 24)

| | | |
|---|---|---|
| $^{1}$H-NMR-Daten ($CDCl_3$): | 1,2 | (d+t, 6H) |
| | 1,3 - 2,6 | (m, 14H) |
| | 2,3 - 3,4 | (m, 6H) |
| | 4,1 | (q, 2H) |
| | 4,6 | (d, 1H) |
| | 5,2 | (s, 2H) |
| | 7,2 | (s, 5H) |
| | 7,4 | (s, 5H) |

Beispiel 25

N-(1-S-Carbethoxy-3-phenyl-propyl)-S-alanyl-1S,4R,10S-dehydrocyclohepta/c_7pyrrol-1-carbonsäurebenzylester (= Diastereomer A 25)

| | | |
|---|---|---|
| $^{1}$H-NMR-Daten ($CDCl_3$): | 1,25 | (d+t, 6H) |
| | 1,3 - 2,5 | (m, 14H) |
| | 2,3 - 3,4 | (m, 6H) |
| | 4,1 | (q, 2H) |
| | 4,5 | (d, 1H) |
| | 5,2 | (s, 2H) |
| | 7,2 | (s, 5H) |
| | 7,4 | (s, 5H) |

Beispiel 26

N-(1-S-Carbethoxy-3-phenyl-propyl)-S-alanyl-1S,4S,8S octahydrocyclopenta/c_7pyrrol-1-carbonsäure-tert.butylester (=Diastereomer A 26)

| | | |
|---|---|---|
| $^{1}$H-NMR-Daten ($CDCl_3$): | 1,25 | (d+t, 6H) |
| | 1,3 | (s, 9H) |
| | 1,3 - 2,6 | (m, 10H) |
| | 2,3 - 3,4 | (m, 6H) |
| | 4,1 | (q, 2H) |

| | |
|---|---|
| 4,6 | (d, 1H) |
| 7,2 | (s, 5H) |

Beispiel 27

N-(1-S-Carbethoxy-3-phenyl-propyl)-S-alanyl-1S,4S,9S-octahydrocyclohexa/c_7pyrrol-1-carbonsäure-tert-butylester (= Diastereomer A 27)

$^1$H-NMR-Daten ($CDCl_3$):

| | |
|---|---|
| 1,25 | (d+t, 6H) |
| 1,3 | (s, 9H) |
| 1,3 - 2,4 | (m, 12H) |
| 2,4 - 3,5 | (m, 6H) |
| 4,1 | (q, 2H) |
| 4,6 | (d, 1H) |
| 7,2 | (s, 5H) |

Beispiel 28

N-(1-S-Carbethoxy-3-phenyl-propyl)-S-alanyl-1S,4R,8S-octahydrocyclopenta/c_7pyrrol-1-carbonsäure-tert.butylester (= Diastereomer A 28)

$^1$H-NMR-Daten ($CDCl_3$):

| | |
|---|---|
| 1,25 | (d+t, 6H) |
| 1,3 | (s, 9H) |
| 1,3 - 3,5 | (m, 10H) |
| 2,5 - 3,5 | (m, 6H) |
| 4,1 | (q, 2H) |
| 4,55 | (d, 1H) |
| 7,2 | (s, 5H) |

Beispiel 29

N-(1-S-Carbethoxy-3-phenyl-propyl)-S-alanyl)-1S,4R,9S-octahydrocyclohexa/c)pyrrol-1-carbonsäure-tert-butylester (=Diastereomer A 29)

| | | |
|---|---|---|
| $^1$H-NMR-Daten ($CDCl_3$): | 1,25 | (d+t, 6H) |
| | 1,3 | (s, 9H) |
| | 1,3 - 2,6 | (m, 12H) |
| | 2,6 - 3,4 | (m, 6H) |
| | 4,1 | (q, 2H) |
| | 4,5 | (d, 1H) |
| | 7,2 | (s, 5H) |

Beispiel 30

N-(1-S-Carbethoxy-3-phenyl-propyl)-S-alanyl-1S,4S,8S-octahydropenta/c_7pyrrol-1-carbonsäure-hydrochlorid

Methode A:

0,7 g Diastereomer A 20 aus Beispiel 20 werden in 25 ml Ethanol mit 100 mg Palladium-Tierkohle (10 %) bei 20 bis 25°C unter Normaldruck hydriert. Nach Abtrennen des Katalysators wird die Lösung mit 0,5 n äthanolischem Chlorwasserstoff bis zur sauren Reaktion versetzt. Die Lösung wird im Vakuum eingeengt und der Rückstand mit Diisopropylether verrieben. Es werden 400 mg der Titelverbindung als amorphes Pulver erhalten.

| | | |
|---|---|---|
| $^1$H-NMR-Daten (DMSO-$d_6$): | 1,25 | (d+t, 6H) |
| | 1,3 - 2,4 | (m, 10H) |
| | 2,4 - 3,7 | (m, 6H) |
| | 4,1 | (q, 2H) |
| | 4,4 | (d, 1H) |
| | 7,2 | (s,5H) |

Methode B:

Eine Lösung von 0,8g Diastereomer A 26 aus Beispiel 26 in 5 ml Methylenchlorid wird mit trockenem Chlorwasserstoff-Gas gesättigt und 16 Stunden bei 20 bis 25°C stehengelassen. Die Lösung wird im Vakuum eingeengt; der Rückstand wird mit Diisopropylether verrieben und abgesaugt.

Ausbeute; 550 mg.

Beispiel 31

N-(1-S-Carbethoxy-3-phenyl-propyl)-S-alanyl-1R,4R,8R-octahydrocyclopenta/c_7pyrrol-1-carbonsäure-hydrochlorid

Diese Verbindung wird aus dem Diastereomer B 20 von Beispiel 20 in einer zur Methode A des Beispiels 30 analogen Verfahrensweise erhalten.
Farblose Kristalle vom Fp. 185 - 188°C

Beispiel 32

N-(1-S-Carbethoxy-3-phenyl-propyl)-S-alanyl-1S,4R,8S-octahydrocyclopenta/c_7pyrrol-1-carbonsäure-hydrochlorid

Diese Verbindung wird aus dem Diastereomeren A 21 des Beispiels 21 in einer zur Methode A des Beispiels 30 analogen Verfahrensweise erhalten.

| | | |
|---|---|---|
| $^1$H-NMR-Daten ($CDCl_3$): | 1,2 | (d+t, 6H) |
| | 1,3 - 2,4 | (m, 10H) |
| | 2,4 - 3,7 | (m, 6H) |
| | 4,1 | (q, 2H) |
| | 4,4 | (d, 1H) |
| | 7,2 | (S, 5H) |

Beispiel 33

N-(1-S-Carbethoxy-3-phenyl-propyl-S-alanyl-1S,4S,9S)-octahydrocyclohexa/c_7pyrrol-1-carbonsäure-hydrochlorid

Diese Verbindung wird aus dem Diastereomeren A 22 des Beispiels 22 in einer zur Methode A des Beispiels 30 analogen Verfahrensweise erhalten.

| | | |
|---|---|---|
| $^{1}$H-NMR-Daten (DMSO-$d_6$): | 1,3 | (d+t, 6H) |
| | 1,3 - 2,4 | (m, 12H) |
| | 2,4 - 3,7 | (m, 6H) |
| | 4,1 | (q, 2H) |
| | 4,4 | (d, 1H) |
| | 7,2 | (s, 5H) |

Beispiel 34

N-(1-S-Carbethoxy-3-phenyl-propyl)-S-alanyl-1S,4R,9S-octahydrohexa/c_7pyrrol-1-carbonsäure-hydrochlorid

Diese Verbindung wird aus dem Diastereomeren A 23 des Beispiels 23 analog der in Beispiel 30 beschriebenen Methode A hergestellt.

| | | |
|---|---|---|
| $^{1}$H-NMR-Daten (DMSO-$d_6$): | 1,3 | d+t, 6H) |
| | 1,3 - 2,4 | (m, 12H) |
| | 2,4 - 3,7 | (m, 6H) |
| | 4,1 | (q, 2H) |
| | 4,45 | (d, 1H) |
| | 7,2 | (s, 5H) |

Die Verbindungen der Beispiele 32 bis 34 können auch aus den Diastereomeren A 26 bis A 28 gemäß der im Beispiel 30 beschriebenen Methode B hergestellt werden.

Beispiel 35

N-(1-S-Carbethoxy-3-phenyl-propyl)-S-alanyl-1S,4S,10S-decahydrocyclohepta/c_7pyrrol-1-carbonsäure-hydrochlorid

Diese Verbindung wird aus dem Diastereomeren A 24 des Beispiels 24 analog der in Beispiel 30 beschriebenen Methode A hergestellt.

| | | |
|---|---|---|
| $^1$H-NMR-Daten (DMSO-$d_6$): | 1,3 | (d+t, 6H) |
| | 1,3 - 2,4 | (m, 14H) |
| | 2,4 - 3,7 | (m, 6H) |
| | 4,1 | (q, 2H) |
| | 4,6 | (d, 1H) |
| | 7,2 | (s, 5H) |

Beispiel 36

N-(1-S-Carbethoxy-3-phenyl-propyl)-S-alanyl-1S,4R,10S-decahydrocyclohepta/c_7pyrrol-1-carbonsäure-hydrochlorid

Diese Verbindung wird aus dem Diastereomeren A 25 des Beispiels 25 analog der im Beispiel 30 beschriebenen Methode A hergestellt.

| | | |
|---|---|---|
| $^1$H-NMR-Daten (DMSO-$d_6$): | 1,3 | (d+t, 6H) |
| | 1,3 - 2,5 | (m, 14H) |
| | 2,5 - 3,7 | (m, 6H) |
| | 4,1 | (q, 2H) |
| | 4,4 | (d, 1H) |
| | 7,2 | (s, 5H) |

Beispiel 37

N-(1-S-Carboxy-3-phenyl-propyl)-S-alanyl-1S,4S,8S-octahydrocyclopenta/c_7pyrrol-1-carbonsäure

Eine Lösung von 1 g N-(1-S-Carbethoxy-3-phenyl-propyl)-S-alanyl-1S,4S,8S-octahydrocyclopenta/c_7pyrrol-1-carbonsäure-hydrochlorid in 10 ml Wasser wird mit zwei Äquivalenten Kaliumhydroxid und einem 10%igen Überschuß 4n-Kaliumhydroxid-Lösung versetzt. Nach 8stündigem Rühren bei 20 bis 25°C wird die Reaktionslösung mit 2n-Salzsäure auf einen pH-Wert von 4,0 gestellt und im Vakuum eingeengt. Der Rückstand wird in Essigester aufgenommen; von abgeschiedenem Salz wird abfiltriert. Die Essigesterlösung wird eingeengt, der Rückstand mit Diisopropylether verrieben und abgesaugt.

Ausbeute: 0,6 g

| | | |
|---|---|---|
| $^1$H-NMR-Daten ($CDCl_3$) | 1,2 | (d, 3H) |
| | 1,2 - 3,8 | (m, 16H) |
| | 4,0 - 4,6 | (m, 1H) |
| | 7,2 | (s, 5H) |

Beispiel 38

N-(1-S-Carboxy-3-phenyl-propyl)-S-alanyl-1S,4R,8S-octahydrocyclopenta/c_/pyrrol-1-carbonsäure

Diese Verbindung wird aus der Verbindung von Beispiel 32 analog dem in Beispiel 37 beschriebenen Verfahren hergestellt.

| | | |
|---|---|---|
| $^1$H-NMR-Daten ($CDCl_3$) | 1,25 | (d, 3H) |
| | 1,3 - 3,9 | (m, 16H) |
| | 4,0 - 4,6 | (m, 1H) |
| | 7,2 | (s, 5H) |

Beispiel 39

N-(1-S-Carboxy-3-phenyl-propyl)-S-alanyl-1S,4S,9S-octahydrocyclohexa/¯c7pyrrol-1-carbonsäure

Diese Verbindung wird aus der Verbindung von Beispiel 33 analog dem in Beispiel 37 beschriebenen Verfahren hergestellt.

| | | |
|---|---|---|
| $^1$H-NMR-Daten ($CDCl_3$) | 1,25 | (d, 3H) |
| | 1,3 - 4,0 | (m, 18H) |
| | 4,0 - 4,6 | (m, 1H) |
| | 7,2 | (s, 5H) |

Beispiel 40

N-(1-S-Carboxy-3-phenyl-propyl)-S-alanyl)-1S,4R,9S-cyclohexa/c 7pyrrol-1-carbonsäure

Diese Verbindung wird aus der Verbindung von Beispiel 34 analog dem in Beispiel 37 beschriebenen Verfahren hergestellt.

| | | |
|---|---|---|
| $^1$NMR-Daten ($CDCl_3$) | 1,3 | (d, 3H) |
| | 1,3 - 4,0 | (m, 18H) |
| | 4,0 - 4,6 | (m, 1H) |
| | 7,2 | (s, 5H) |

Beispiel 41

N-(1-S-Carboxy-3-phenyl-propyl)-S-alanyl-1S,4S,10S-decahydrocyclohepta/c 7pyrrol-1-carbonsäure

Diese Verbindung wird aus der Verbindung von Beispiel 35 analog dem in Beispiel 37 beschriebenen Verfahren hergestellt.

| | | |
|---|---|---|
| $^1$H-NMR-Daten ($CDCl_3$) | 1,2 | (d, 3H) |
| | 1,3 - 4,0 | (m, 20H) |
| | 4,0 - 4,5 | (m, 1H) |
| | 7,2 | (s, 5H) |

## Beispiel 42

**N-(1-S-Carboxy-3-phenyl-propyl)-S-alanyl-1S,4R,10S-decahydrocyclohepta/c_7pyrrol-1-carbonsäure**

Diese Verbindung wird aus der Verbindung von Beispiel 36 analog dem in Beispiel 37 beschriebenen Verfahren hergestellt.

| | | |
|---|---|---|
| $^1$H-NMR-Daten ($CDCl_3$) | 1,2 | (d, 3H) |
| | 1,3 - 4,0 | (m, 20H) |
| | 4,0 - 4,5 | (m, 1H) |
| | 7,2 | (s, 5H) |

## Beispiel 43

**N-(1-S-Carbethoxy-3-phenyl-3-oxo-propyl)-S-alanyl-1S,4S,8S-octahydrocyclopenta/c_7pyrrol-1-carbonsäurebenzylester**

2,5 g N-(1-S-Carbethoxy-3-phenyl-3-oxo-propyl)-S-alanin werden zusammen mit 1,2 g 1-Hydroxybenztriazol, 2,5 g (±)-1S,4S,8S-Octahydrocyclopenta/c_7pyrrol-1-carbonsäurebenzylester-hydrochlorid , 1,25 ml N-Ethylmorpholin und 2 g Dicyclohexylcarbodiimid in 20 ml Dimethylformamid gegeben. Man rührt 1 Stunde bei 0°C und anschließend 12 Stunden bei 20 bis 25°C.

Die Reaktionslösung wird mit 25 ml Essigester verdünnt; abgeschiedener Harnstoff wird abgesaugt. Nach dem Einengen im Vakuum wird der erhaltene Rückstand in Ether aufgenommen

die etherische Lösung mit gesättigtem wäßrigen Natriumbicarbonat und mit Wasser gewaschen, getrocknet und eingedampft. Man erhält 3 g der Titelverbindung als Diastereomerengemisch.

| | | |
|---|---|---|
| $^1$H-NMR-Daten ($CDCl_3$) | 1,25 | (d+t, 6H) |
| | 1,0 - 2,8 | (m, 14H) |
| | 3,4 - 5,1 | (m, 6H) |
| | 7,2 - 8,2 | (m, 10H) |

Das Diastereomerengemisch kann über Kieselgel mit Cyclohexan-Essigester als Elutionsmittel in die optisch reinen Verbindungen getrennt werden.

## Beispiele 44 bis 50

Die nachfolgenden Verbindungen der Beispiele 44 bis 50 lassen sich in einer zu Beipiel 43 analogen Verfahrensweise unter Verwendung der entsprechenden bicyclischen Carbonsäureester-Verbindungen herstellen.

## Beispiel 44

N-(1-S-Carbethoxy-3-phenyl-3-oxo-propyl)-S-alanyl-1S,4R,8S-Octahydrocyclopenta/c_7pyrrol-1-carbonsäure-benzylester

| | | |
|---|---|---|
| $^1$H-NMR-Daten ($CDCl_3$) | 1,25 | (d+t, 6H) |
| | 1,3 - 2,4 | (m, 6H) |
| | 2,4 - 3,8 | (m, 8H) |
| | 4,2 | (q, 2H) |
| | 4,3 - 4,8 | (m, 1H) |
| | 5,2 | (s, 2H) |
| | 7,2 | (s, 5H) |
| | 7,4 - 8,1 | (m, 5H) |

Beispiel 45

N-(1-S-Carbethoxy-3-phenyl-3-oxo-propyl)-S-alanyl-1S,4S,9S-octahydrocyclohexa/c_7pyrrol-1-carbonsäure-benzylester

| | | |
|---|---|---|
| $^1$H-NMR-Daten ($CDCl_3$) | 1,2 | (d, 3H) |
| | 1,3 | (t, 3H) |
| | 1,3 - 2,4 | (m, 8H) |
| | 2,4 - 3,8 | (m, 8H) |
| | 4,1 | (q, 2H) |
| | 4,3 - 4,8 | (m, 1H) |
| | 5,2 | (s, 2H) |
| | 7,2 - 8,2 | (m, 10H) |

Beispiel 46

N-(1-S-Carbethoxy-3-phenyl-3-oxo-propyl)-S-alanyl-1S,4R,9S-cyclohexa/c_7pyrrol-1-carbonsäure-benzylester

| | | |
|---|---|---|
| $^1$H-NMR-Daten ($CDCl_3$) | 1,25 | (d+t, 6H) |
| | 1,3 - 2,4 | (m, 8H) |
| | 2,4 - 3,8 | (m, 8H) |
| | 4,1 | (q, 2H) |
| | 4,3 - 4,8 | (m, 2H) |
| | 5,2 | (s, 2H) |
| | 7,1 - 8,2 | (m, 10H) |

Beispiel 47

N-(1-S-Carbethoxy-3-phenyl-3-oxo-propyl)-S-alanyl-1S,4S,8S-cyclopenta/c_7pyrrol-1-carbonsäure-tert.-butylester

| | | |
|---|---|---|
| $^1$H-NMR-Daten ($CDCl_3$) | 1,25 | (d+t, 6H) |
| | 1,3 | (s, 9H) |
| | 1,3 - 2,4 | (m, 6H) |

| | | |
|---|---|---|
| | 2,4 - 3,8 | (m, 8H) |
| | 4,1 | (q, 2H) |
| | 4,3 - 4,8 | (m, 1H) |
| | 7,2 - 8,0 | (m, 5H) |

Beispiel 48

N-(1-S-Carbethoxy-3-phenyl-3-oxo-propyl)-S-alanyl-1S,4R,8S-octahydrocyclopenta/c_7pyrrol-1-carbonsäure-tert.-butylester

| | | |
|---|---|---|
| $^1$H-NMR-Daten ($CDCl_3$) | 1,3 | (d+t, 6H) |
| | 1,35 | (s, 9H) |
| | 1,4 - 2,5 | (m, 6H) |
| | 2,5 - 3,8 | (m, 8H) |
| | 4,15 | (q, 2H) |
| | 4,3 - 4,8 | (m, 1H) |
| | 7,2 - 8,0 | (m, 5H) |

Beispiel 49

N-(1-S-Carbethoxy-3-phenyl-3-oxo-propyl)-S-alanyl-1S,4S,9S-octahydrocyclohexa/c_7pyrrol-1-carbonsäure-tert.-butylester

| | | |
|---|---|---|
| $^1$H-NMR-Daten ($CDCl_3$) | 1,3 | (d+t, 6H) |
| | 1,4 | (s, 9H) |
| | 1,4 - 2,5 | (m, 8H) |
| | 2,5 - 3,8 | (m, 8H) |
| | 4,2 | (q, 2H) |
| | 4,3 - 4,8 | (m, 1H) |
| | 7,3 - 8,1 | (m, 5H) |

Beispiel 50

N-(1-S-Carbethoxy-3-phenyl-3-oxo-propyl)-S-alanyl-1S,4R,9S-octahydrocyclohexa/c_7pyrrol-1-carbonsäure-tert.-butylester

| | | | |
|---|---|---|---|
| $^1$H-NMR-Daten | ($CDCl_3$) | 1,2 | (d+t, 6H) |
| | | 1,3 | (s, 9H) |
| | | 1,4 - 2,6 | (m, 8H) |
| | | 2,6 - 3,9 | (m, 8H) |
| | | 4,2 | (q, 2H) |
| | | 4,3 - 4,8 | (m, 1H) |
| | | 7,3 - 8,1 | (m, 5H) |

Beispiel 51

N-(1-S-Carbethoxy-3-phenyl-3-oxo-propyl)-S-alanyl-1S,4S,8S-octahydrocyclopenta/c_7pyrrol-1-carbonsäure

0,9 g der Verbindung des Beispiels 47 werden in 6 ml Trifluoressigsäure 2 Stunden bei 20°C gerührt. Man engt im Vakuum ein, verreibt mit Diisopropylether und saugt ab.

Ausbeute: 0,4 g

| | | | |
|---|---|---|---|
| $^1$H-NMR-Daten | ($CDCl_3$) | 1,2 | (d+t, 6H) |
| | | 1,3 - 3,6 | (m, 12H) |
| | | 4,2 | (q, 2H) |
| | | 4,1 - 4,6 | (m, 4H) |
| | | 7,3 - 8,1 | (m, 5H) |

Analog der in Beispiel 51 beschriebenen Verfahrensweise können die Verbindungen der nachfolgenden Beispiele 52 bis 54 hergestellt werden:

Beispiel 52

N-(1-S-Carbethoxy-3-phenyl-3-oxo-propyl)-S-alanyl-1S,4R,8S-octahydrocyclopenta/c_7pyrrol-1-carbonsäure

| | | |
|---|---|---|
| $^1$H-NMR-Daten ($CDCl_3$): | 1,25 | (d+t, 6H) |
| | 1,3 - 3,6 | (m, 12H) |
| | 4,2 | ( , 2H) |
| | 4,1 - 4,6 | (m, 4H) |
| | 7,3 - 8,1 | (m, 5H) |

Beispiel 53

N-(1-S-Carbethoxy-3-phenyl-3-oxo-propyl)-S-alanyl-1S,4S,9S-octahydrocyclohexa/c7pyrrol-1-carbonsäure

| | | |
|---|---|---|
| $^1$H-NMR-Daten ($CDCl_3$): | 1,3 | (d+t, 6H) |
| | 1,3 - 3,6 | (m, 14H) |
| | 4,2 | (q, 2H) |
| | 4,1 - 4,6 | (m, 4H) |
| | 7,3 - 8,1 | (m, 5H) |

Beispiel 54

N-(1-S-Carbethoxy-3-phenyl-3-oxo-propyl)-S-alanyl-1S,4R,9S-octahydrocyclohexa/c_7pyrrol-1-carbonsäure

| | | |
|---|---|---|
| $^1$H-NMR-Daten ($CDCl_3$) | 1,3 | (d+t, 6H) |
| | 1,3 - 3,6 | (m, 14H) |
| | 4,2 | (q, 2H) |
| | 4,1 - 4,6 | (m, 4H) |
| | 7,3 - 8,1 | (m, 5H) |

Beispiel 55

N-(1-S-Carboxy-3-phenyl-3-oxo-propyl)-S-alanyl-1S,4S,8S-octahydrocyclopenta/c_7pyrrol-1-carbonsäure

Zur Herstellung dieser Verbindung werden 0,5 g N-(1-S-Carbethoxy-3-phenyl-3-oxo-propyl)-S-alanyl-1S,4S,8S-octahydrocyclopenta/c_7pyrrol-1-carbonsäure analog der in Beispiel 37 beschriebenen Verfahrensweise mit Kaliumhydroxid umgesetzt:

| | | |
|---|---|---|
| $^1$H-NMR-Daten ($CDCl_3$): | 1,2 | (d, 3H) |
| | 1,2 - 3,8 | (m, 14H) |
| | 4,0 - 4,6 | (m, 1H) |
| | 7,2 - 7,9 | (m, 5H) |

Beispiel 56

N-(1-S-Carbethoxy-3-phenyl-3-hydroxy-propyl)-S-alanyl-1S,4S,8S-octahydrocyclopenta/c_7pyrrol-1-carbonsäure

1 g N-(1-S-Carbethoxy-3-phenyl-3-oxo-propyl)-S-alanyl-1S,4S,8S-octahydrocyclopenta/c_7pyrrol-1-carbonsäure werden in 50 ml wasserfreiem Ethanol gelöst und mit 50 mg Palladium/Kohle bei 20 bis 25°C unter Normaldruck mit 1 Mol Äquivalent Wasserstoff hydriert. Nach Abfiltrieren des Katalysators wird die Lösung eingedampft, der Rückstand mit Diisopropyläther verrieben und abgesaugt.
Ausbeute: 0,7 g

| | | |
|---|---|---|
| $^1$H-NMR-Daten ($CDCl_3$): | 1,3 | (d+t, 6H) |
| | 1,3 - 3,6 | (m, 12H) |
| | 4,2 | (q, 2H) |
| | 4,1 - 4,6 | (m, 4H) |
| | 4,8 | (d, 1H) |
| | 7,0 - 7,5 | (m, 5H) |

**Beispiel 57**

**S-Alanyl-1S,4S,8S-octahydrocyclopenta/c_7pyrrol-1-carbonsäure-benzylester**

a) N-tert.-Butoxycarbonyl-S-alanyl-1S,4S,8S-octahydrocyclopenta/c_7pyrrol-1-carbonsäure-benzylester

Zu einer Lösung von 19 g BOC-Ala-OH in 100 ml DMF werden 13 ml N-Ethylmorpholin, 13,5 g 1-Hydroxy-benzotriazol und 29,6 g 1S,4S,8S-Octahydrocyclopenta/c_7pyrrol-1-carbonsäure-benzylester-Hydrochlorid gegeben. Das Gemisch wird im Eisbad gekühlt und mit 21 g Dicyclohexylcarbodiimid versetzt. Man rührt 15 Stunden bei 20 bis 25°C. Der ausgefallene Harnstoff wird abgesaugt, das Filtrat im Vakuum eingeengt und in Essigester aufgenommen. Die organische Phase wird je 3 mal mit wäßrigem Kaliumhydrogensulfat, Kaliumhydrogencarbonat und Natriumchlorid extrahiert, getrocknet und eingedampft. Der Rückstand wird an Kieselgel mit Essigester/Cyclohexan (1 : 3) chromatographiert. Die erste Fraktion enthält das gewünschte Produkt.
Ausbeute: 21 g

| | | |
|---|---|---|
| $^1$H-NMR-Daten ($CDCl_3$): | 1,3 | (d, 3H) |
| | 1,45 | (s, 9H) |
| | 1,1 - 2,4 | (m, 8H) |
| | 3,2 - 3,9 | (m, 4H) |
| | 5,3 | (s, 2H) |
| | 7,4 | (s, 5H) |

b) S-Alanyl-1S,4S,8S-octahydrocyclopenta/c_7pyrrol-1-carbonsäure-benzylester

20,5 g N-tert.Butoxycarbonyl-S-alanyl-1S,4S,8S-octahydrocyclopenta/c_7pyrrol-1-carbonsäure-benzylester

werden in 50 ml Trifluoressigsäure gelöst. Nach einer Reaktionszeit von 10 Min. engt man die Lösung im Vakuum ein, digeriert den Rückstand mehrfach mit Diisopropylether und trocknet ihn im Vakuum.
Ausbeute: 12,5 g

Beispiele 58 bis 62

Diese Verbindungen werden analog den in Beispiel 57 unter a) und b) beschriebenen Verfahrensweisen hergestellt:

Beispiel 58

S-Alanyl-1S,4R,8S-octahydrocyclopenta/c_7pyrrol-1-carbonsäure-benzylester

| | | |
|---|---|---|
| $^1$H-NMR-Daten ($CDCl_3$): | 1,3 | (d, 3H) |
| | 1,1 - 2,4 | (m, 8H) |
| | 3,2 - 3,9 | (m, 4H) |
| | 5,3 | (s, 2H) |
| | 7,4 | (s, 5H) |

Beispiel 59

S-Alanyl-1S,4S,9S-octahydrocyclohexa/c_7pyrrol-1-carbonsäure-benzylester

| | | |
|---|---|---|
| $^1$H-NMR-Daten ($CDCl_3$): | 1,3 | (d, 3H) |
| | 1,3 - 2,6 | (m, 10H) |
| | 3,2 - 3,9 | (m, 4H) |
| | 5,3 | (s, 2H) |
| | 7,4 | (s, 5H) |

Beispiel 60

S-Alanyl-1S,4R,9S-octahydrocyclohexa/c_7pyrrol-1-carbon-säure-benzylester

| | | |
|---|---|---|
| $^1$H-NMR-Daten (CDCl$_3$): | 1,3 | (d, 3H) |
| | 1,3 - 2,5 | (m, 10H) |
| | 3,1 - 3,9 | (m, 4H) |
| | 5,2 | (s, 2H) |
| | 7,4 | (s, 5H) |

Beispiel 61

S-Alanyl-1S,4S,10S-decahydrocyclohepta/c_7pyrrol-1-carbon-säure-benzylester

| | | |
|---|---|---|
| $^1$H-NMR-Daten (CDCl$_3$) | 1,3 | (d, 3H) |
| | 1,3 - 2,7 | (m, 12H) |
| | 3,1 - 3,9 | (m, 4H) |
| | 5,2 | (s, 2H) |
| | 7,4 | (s, 5H) |

Beispiel 62

S-Alanyl-1S.4R,10S-decahydrocyclohepta/c_7pyrrol-1-carbon-säure-benzylester

| | | |
|---|---|---|
| $^1$H-NMR-Daten (CDCl$_3$) | 1,3 | (d, 3H) |
| | 1,3 - 2,8 | (m, 12H) |
| | 3,1 - 3,9 | (m, 4H) |
| | 5,2 | (s, 2H) |
| | 7,4 | (s, 5H) |

Beispiel 63

N-(1-S-Carbethoxy-3-phenyl-propyl)-S-alanyl-1S,4S,8S-octahydrocyclopenta/c_7pyrrol-1-carbonsäure

a) N-(1-S-Carbethoxy-3-phenyl-propyl)-S-alanyl-1S,4S,8S-octahydrocyclopenta/c_7pyrrol-1-carbonsäure-benzylester

10 m Mol S-Alanyl-1S,4S,8S-octahydrocyclopenta/c_7pyrrol-1-carbonsäure-benzylester werden in 30 ml wasserfreiem Ethanol gelöst. Man stellt die Lösung mittels ethanolischem Kaliumhydroxid auf einen pH-Wert von 7,0 und gibt 1 g pulverisiertes Molekularsieb (4Å) und anschließend 10 mMol 2-Keto-4-phenyl-buttersäureäthylester hinzu. Es wird eine Lösung von 1 g Natriumcyanborhydrid in 10 ml wasserfreiem Ethanol langsam zugetropft. Nach einer Reaktionszeit von 20 Stunden bei 20 bis 25°C wird die Reaktionslösung filtriert und das Lösungsmittel abdestilliert. Der Rückstand wird in Essigester/Wasser aufgenommen. Nach Eindampfen der Essigesterphase wird der Rückstand an Kieselgel mit Essigester/Cyclohexan (1 : 4) chromatographiert. Die $^1$H-NMR-Daten stimmen mit den Daten der Verbindung aus Beispiel 20 überein.

b) Die oben erhaltene Verbindung wird wie in Beispiel 30, Methode A beschrieben zur gewünschten Verbindung weiter umgesetzt.

Beispiel 64

N-(1-S-Carbethoxy-3-phenyl-3-oxo-propyl)-S-alanyl-1S,4S,9S-octahydrocyclohexa/c_7pyrrol-1-carbonsäure-benzylester

10 mMol S-Alanyl-1S,4S,9S-octahydrocyclopenta/c_7pyrrol-

1-carbonsäure-benzylester werden zusammen mit 10 mMol 3-Benzoyl-acrylsäureethylester und 10 mMol Triethylamin in 100 ml wasserfreiem Ethanol gelöst und das Gemisch 24 Stunden bei 20 bis 25°C gerührt. Man neutralisiert mit 1 n Salzsäure, dampft zur Trockene ein und nimmt den Rückstand mit Essigester/Wasser auf. Die Essigesterphase wird getrocknet, eingedampft und am Kieselgel chromatographiert.

Beispiel 65

N-(1-S-Carbethoxy-3-oxo-3-phenyl-propyl)-1S,4S,10S-decahydrocyclohepta/¯c_7pyrrol-1-carbonsäure-benzylester

10 mMol Acetophenon, 10 mMol Glyoxylsäureethylester und 10 mMol S-Alanyl-1S,4S,10S-decahydrocyclohepta/¯c_7pyrrol-1-carbonsäure-benzylester werden in 30 ml Eisessig 36 Stunden auf 45°C erhitzt. Nach Einengen im Vakuum wird mit wäßrigem Natriumbicarbonat alkalisch gestellt und mit Essigester extrahiert. Die Essigesterphase wird eingeengt und an Kieselgel chromatographiert.

Beispiel 66

N-(1-S-Carbethoxy-3-phenyl-propyl)-O-ethyl-S-tyrosyl-1S,4S,8S-octahydrocyclopenta/¯c_7pyrrol-1-carbonsäure

a) N-(1-R,S-Carbethoxy-3-phenyl-propyl)-O-ethyl-S-tyrosyl-benzylester

Man setzt 24 g Benzoylacrylsäure-ethylester in 100 ml Ethanol mit 30 g O-Ethyl-S-tyrosin-benzylester in Gegenwart von 0,5 ml Triethylamin um und erhält nach Einengen der Lösung und Digerieren des Rückstandes mit Diethylether/Petrolether (1:1) und Trocknen im Vakuum 42 g der Titelverbindung.

b) N-(1-R,S-Carbethoxy-3-phenyl-propyl)-O-ethyl-S-tyrosin

40 g der nach Beispiel 66a erhaltenen Verbindung werden in 800 ml Eisessig mit 4 g Pd/C (10 %) bei 100 bar und Raumtemperatur hydriert. Nach Chromatographie an Kieselgel mit Essigester/Cyclohexan (1:3) als Laufmittel und Trocknen des Eindampfrückstandes erhält man 25 g dünnschichtchromatographisch nahezu einheitliche Titelverbindung, Schmp. 205-213°C.

| | | | | |
|---|---|---|---|---|
| $C_{22}H_{29}NO_5$ (399,5) | Ber. | C 69,15 | H 7,31 | N 3,50 |
| | Gef. | C 69,5 | H 7,4 | N 3,3 |

c) N-(1-S-Carbethoxy-3-phenylpropyl)-O-ethyl-S-tyrosyl-1S,4S,8S-octahydrocyclopenta/¯c 7pyrrol-1-carbonsäure

Man setzt analog der in Beispiel 20 beschriebenen Verfahrensweise 1,5 g des in Beispiel 10 beschriebenen Benzylesters mit 2,5g N-(1-R,S-Carbethoxy-3-phenyl-propyl)-O-ethyl-S-tyrosin, 1,3g Dicyclohexylcarbodiimid und 0,8 g 1-Hydroxybenztriazol um. Chromatographie des Rohprodukts am Kieselgel mit Cyclohexan/Essigester (1:1) als Laufmittel ergibt 1 g der Titelverbindung als farbloses Öl.

Die $^1$H-NMR-Daten und das Massenspektrum sind im Einklang mit der angegebenen Struktur.

Der Benzylester wird analog der in Beispiel 30 beschriebenen Verfahrensweise hydrogenolysiert. Man erhält 0,6 g der Titelverbindung als farbloses amorphes Pulver.

$^1$H-NMR-Daten: 7,3 (s, 5H);
7,1-6,5 (2d, 4H);
4,4-4,0 (m, 4H);
3,9-3,0 (m, 4H);
2,9-1,2 (m, 15H);
1,4 (t, 3H);
1,25 (t, 3H).

Beispiel 67

N-(1-S-Carbethoxy-3-phenyl-propyl)-O-methyl-S-tyrosyl-1S,4S,8S-octahydrocyclopenta/¯c_7pyrrol-1-carbonsäure

Man erhält die Titelverbindung analog der in Beispiel 66 beschriebenen Verfahrensweise unter Verwendung von O-Methyl-tyrosin-benzylester anstelle von O-Ethyl-tyrosin-benzylester in der 66a) analogen Stufe.

$^{1}$H-NMR-Daten: 7,2 (s, 5H);
7,1-6,5 (2d, 4H);
4,4-4,0 (m, 3H);
3,9-3,0 (m, 3H);
4,5 (s, 3H);
2,9-1,2 (m, 15 H);
1,3 (t, 3H).

Beispiel 68

N-(1-S-Carbethoxy-3-phenyl-propyl)-O-ethyl-S-tyrosyl-1S,4S,9S-octahydroisoindol-1-carbonsäure

Hergestellt analog der in Beispiel 66 beschriebenen Verfahrensweise aus der in Beispiel 2 beschriebenen Aminosäure. Die $^{1}$H-NMR-Daten stimmen mit der angegebenen Struktur überein.

Beispiel 69

N-(1-S-(Carbethoxy-3-phenyl-propyl)-O-methyl-S-tyrosyl-1S,4S,9S-octahydroisoindol-2-carbonsäure

Hergestellt analog der in Beispiel 68 beschriebenen Verfahrensweise unter Verwendung der in Beispiel 2 beschriebenen Aminosäure. Die $^{1}$H-NMR-Daten stimmen mit der angegebenen Struktur überein.

Beispiel 70

N-(1-S-Carbethoxy-3-phenyl-propyl)-O-ethyl-S-tyrosyl 1S,4S,8R-octahydrocyclopenta/¯c 7pyrrol-2-carbonsäure

Hergestellt analog der in Beispiel 66 beschriebenen Verfahrensweise unter Verwendung der in Beispiel 7 beschriebenen Aminosäure.

$^1$H-NMR-Daten: 7,3 (s, 5H);
7,2-6,6 (2d, 4H);
4,4-3,9 (m, 4H);
3,9-3,0 (m, 4H);
2,9-1,2 (m, 15H);
1,35 (t, 3H);
1,25 (t, 3H):

Beispiel 71

N-(1-S-Carbethoxy-3-phenyl-propyl)-O-methyl-S-tyrosyl-1S,4S,8R-octahydrocyclopenta /¯c 7pyrrol-1-carbonsäure

Hergestellt analog der in Beispiel 67 beschriebenen Verfahrensweise unter Verwendung der in Beispiel 7 beschriebenen Aminosäure. Die $^1$H-NMR-Daten stimmen mit der angegebenen Struktur überein.

Beispiel 72

N-(1-S-Carbethoxy-3-phenyl-propyl)-O-ethyl-S-tyrosyl-1S,4S,9R-octahydrocyclohexa/¯c 7pyrrol-1-carbonsäure

Hergestellt analog der in Beispiel 66 beschriebenen Verfahrensweise. Die analytischen Daten stimmen mit der angegebenen Struktur überein.

Beispiel 73

N-(1-S-Carbethoxy-3-phenyl-propyl)-O-methyl-S-tyrosyl-1S,4S,9R-octahydrocyclohexa/¯c ̲7pyrrol-1-carbonsäure

Hergestellt analog der in Beispiel 67 beschriebenen Verfahrensweise. Die analytischen Daten stimmen mit der angegebenen Struktur überein.

Patentansprüche:

1. Verbindungen der Formel I

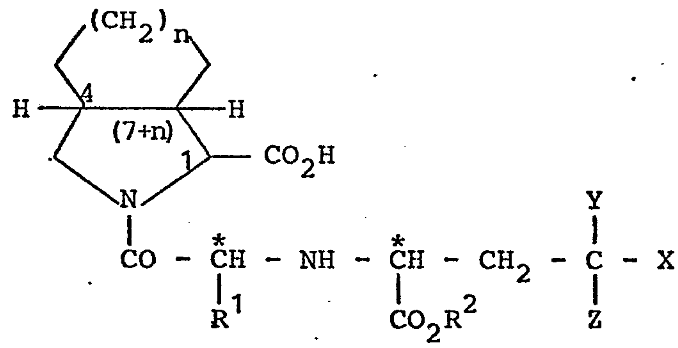

(I)

in der die Wasserstoffatome an den Brückenkopf-C-Atomen 4 und (7+n) zueinander cis- oder trans-konfiguriert sind, wobei die Carboxygruppe am C-Atom 1 zum Wasserstoffatom am C-Atom (7+n) cis- oder transständig orientiert sein kann und worin

n = 1, 2 oder 3,

$R^1$ = Wasserstoff, $(C_1-C_6)$-Alkyl, das gegebenenfalls durch Amino, $(C_1-C_4)$-Acylamino oder Benzoylamino substituiert sein kann, $(C_2-C_6)$-Alkenyl, $(C_5-C_9)$-Cycloalkyl, $(C_5-C_9)$-Cycloalkenyl, $(C_5-C_7)$-Cycloalkyl-$(C_1-C_4)$-alkyl, $(C_6-C_{10})$-Aryl oder teilhydriertes $(C_6-C_{10})$-Aryl, das jeweils durch $(C_1-C_4)$-Alkyl, $(C_1-C_2)$-Alkoxy oder Halogen substituiert sein kann, $(C_6-C_{10})$-Aryl-$(C_1-C_4)$-alkyl, das wie vorstehend definiert im Aryl-Rest substituiert sein kann, einen mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden Aminosäure,

$R^2$ = Wasserstoff, $(C_1-C_6)$-Alkyl, $(C_2-C_6)$-Alkenyl oder $(C_6-C_{10})$-Aryl-$(C_1-C_4)$-alkyl,

Y = Wasserstoff oder Hydroxy,

Z = Wasserstoff oder

Y und Z = zusammen Sauerstoff und

X = $(C_1-C_6)$-Alkyl, $(C_2-C_6)$-Alkenyl, $(C_5-C_9)$-Cycloalkyl, $(C_6-C_{10})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkyl-amino oder Methylendioxy mono-, di- oder trisubstituiert sein kann, oder Indol-3-yl bedeuten,

sowie deren physiologisch unbedenkliche Salze.

2. Verbindungen der Formel I gemäß Anspruch 1, dadurch gekennzeichnet, daß das C-Atom in Position 1 des bicyclischen Ringsystems sowie die mit einem Stern markierten C-Atome der Seitenkette S-Konfiguration aufweisen.

3. Verbindungen der Formel I gemäß einem der Ansprüche 1 oder 2, dadurch gekennzeichnet, daß

$R^1$ = Wasserstoff, $(C_1-C_3)$-Alkyl, $(C_2-C_3)$-Alkenyl, Benzyl, 4-Amino-butyl, 4-Methoxybenzyl oder 4-Ethoxybenzyl,

$R^2$ = Wasserstoff, $(C_1-C_4)$-Alkyl oder Benzyl,

X = Phenyl, das durch $(C_1-C_2)$-Alkyl, $(C_1-C_2)$-Alkoxy, Hydroxy, Fluor, Chlor, Brom, Amino, $(C_1-C_4)$-Alkylamino, Di-$(C_1-C_4)$alkyl-amino, Nitro oder Methylendioxy mono- oder disubstituiert oder im Falle von Methoxy, trisubstituiert sein kann, bedeuten.

4. Verbindungen der Formel I gemäß einem der Ansprüche 1 bis 3, dadurch gekennzeichnet, daß n = 1 oder 2, $R^1$ = Methyl *) und X = Phenyl bedeuten.

*) 4-Methoxybenzyl oder 4-Ethoxybenzyl

5. Verbindungen der Formel I gemäß einem der Ansprüche 1 bis 4, dadurch gekennzeichnet, daß

$R^2$ = Wasserstoff oder Ethyl bedeutet.

6. Verfahren zur Herstellung der Verbindungen der Formel I gemäß einem der Ansprüche 1 bis 5, dadurch gekennzeichnet,

a) daß man eine Verbindung der Formel II,

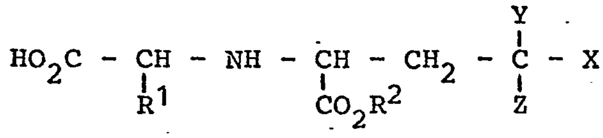

(II)

worin $R^1$, $R^2$, X, Y und Z die Bedeutungen wie in Formel I haben, mit einer Verbindung der Formel III,

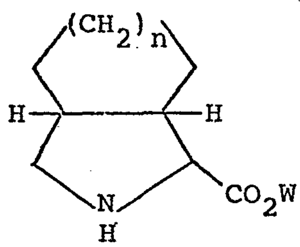

(III)

in der die Konfiguration des bicyclischen Ringsystems und des Substituenten am C-Atom 1 die gleiche ist wie in Formel I und worin

n = 1, 2 oder 3 und

W = einen hydrogenolytisch oder sauer abspaltbaren Rest bedeuten, umsetzt und anschließend den Rest W und gegebenenfalls den Rest $R^2$ unter Bildung der freien Carboxygruppen abspaltet,

b) daß man zur Herstellung der Verbindungen der Formel I, in der Y und Z zusammen Sauerstoff bedeuten,

$b_1$) eine Verbindung der Formel IV,

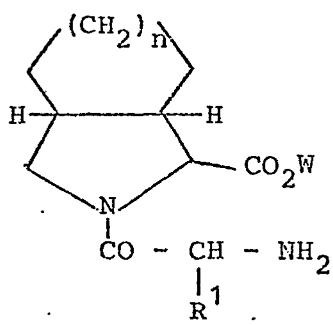

(IV)

in der die Konfiguration des bicyclischen Ringsystems und des Substituenten am C-Atom 1 die gleiche ist

und worin n und $R^1$ die Bedeutungen wie in Formel I und W die Bedeutung wie in Formel III besitzen, mit einer Verbindung der Formel V,

$$R^2O_2C - CH = CH - CO - X \qquad (V)$$

worin $R^2$ und X die Bedeutungen wie in Formel I haben, umsetzt und anschließend den Rest W und gegebenenfalls auch den Rest $R^2$ unter Bildung der freien Carboxygruppen abspaltet, oder

$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$$OHC{-}CO_2R^2 \qquad\qquad X{-}CO{-}CH_3$$

(VI) (VII)

worin X die Bedeutung wie in Formel I hat, umsetzt und anschließend den Rest W und gegebenenfalls den Rest $R^2$ unter Bildung der freien Carboxygruppen abspaltet, oder

c) daß man zur Herstellung von Verbindungen der Formel I, in der Y und Z jeweils Wasserstoff bedeuten,

$c_1$) eine Verbindung der unter $b_1$) genannten Formel IV mit einer Verbindung der Formel VIII,

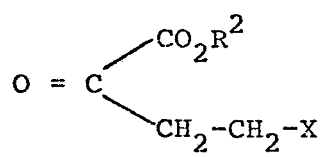 (VIII)

worin $R^2$ und X die Bedeutungen wie in Formel I besitzen, umsetzt, die erhaltenen Schiff-Basen reduziert und anschließend den Rest W und gegebenenfalls den Rest $R^2$ unter Bildung der freien

Carboxygruppen abspaltet, oder

$c_2$) eine Verbindung der Formel I, in der Y und Z zusammen Sauerstoff bedeuten, reduziert, oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, reduziert und die nach a)-d) erhaltenen Verbindungen der Formel I gegebenenfalls in ihre physiologisch unbedenklichen Salze überführt.

7. Verwendung einer Verbindung gemäß einem der Ansprüche 1 bis 5 als Heilmittel.

8. Mittel, enthaltend eine Verbindung gemäß einem der Ansprüche 1 bis 5.

9. Verbindungen der Formel III'

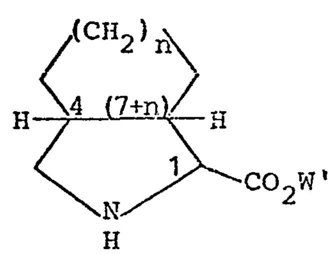

(III')

in der die C-Atome 1, 4 und (7+n) dieselbe Konfiguration wie in Formel III besitzen und worin

n = 1, 2 oder 3

W' = Wasserstoff oder ein hydrogenolytisch oder sauer abspaltbaren Rest bedeuten.

10. Verfahren zur Herstellung der Verbindungen der Formel III' gemäß Anspruch 9, dadurch gekennzeichnet,

a) daß man eine Verbindung der Formel IX,

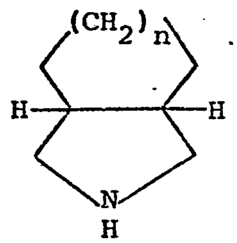

(IX)

worin die H-Atome an den C-Atomen 4 und (7+n) zueinander cis- oder trans-konfiguriert sind und worin n die Zahl 1, 2 oder 3 bedeutet, acyliert, anschließend mit einem Alkohol in Gegenwart eines Leitsalzes zu einer Verbindung der Formel X,

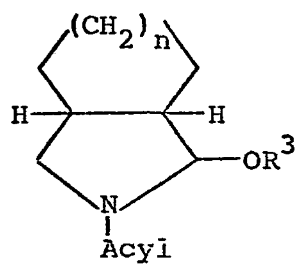

(X)

worin n die oben genannte Bedeutung hat und $R^3$ = $(C_1-C_4)$-Alkyl bedeutet, anodisch oxydiert, diese mit Trimethylsilylcyanid in Gegenwart einer Lewis-Säure zu einer Verbindung der Formel XI,

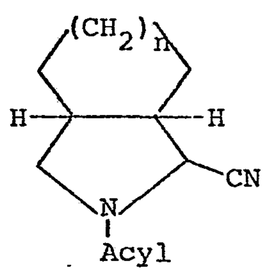

(XI)

worin die H-Atome an den C-Atomen 4 und (7+n) zueinander cis- oder trans-konfiguriert sind und wobei die CN-Gruppe sich in cis-Stellung zum H-Atom am C-Atom (7+n) befindet und worin n die oben genannte Bedeutung hat, umsetzt und diese durch Einwirkung von Säuren

oder Basen zu einer Verbindung der Formel III' mit W' = Wasserstoff hydrolysiert und letztere gegebenenfalls verestert, oder

b) daß man eine Verbindung der Formel XII,

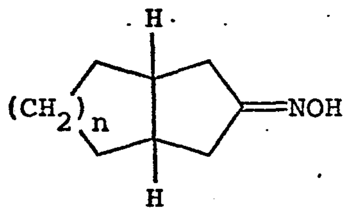

(XII)

worin die H-Atome an den C-Atomen 4 und (7+n) cis- oder trans-konfiguriert sind und n die oben genannnte Bedeutung besitzt in einer Beckmann-Umlagerung zu einer Verbindung der Formel XIII

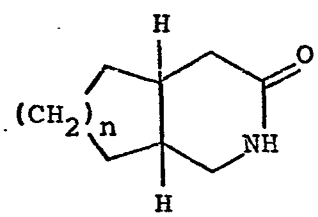

(XIII)

worin n die obengenannte Bedeutung besitzt, umsetzt, diese zu einer Verbindung der Formel XIV,

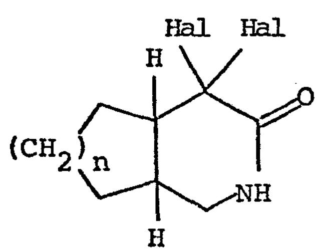

(XIV)

worin n die obengenannte Bedeutung besitzt und Hal ein Halogenatom bedeutet, halogeniert, diese zu einer Verbindung der Formel XV,

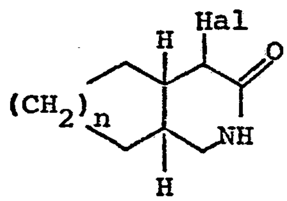

(XV)

worin n und Hal die oben genannten Bedeutungen besitzen, reduziert, und diese anschließend unter Einwirkung einer Base zu einer Verbindung der Formel III' mit W' = Wasserstoff umsetzt und diese gegebenenfalls verestert.

0090362



Patentansprüche für den Vertragsstaat Österreich:

1. Verfahren zur Herstellung von Verbindungen der Formel I

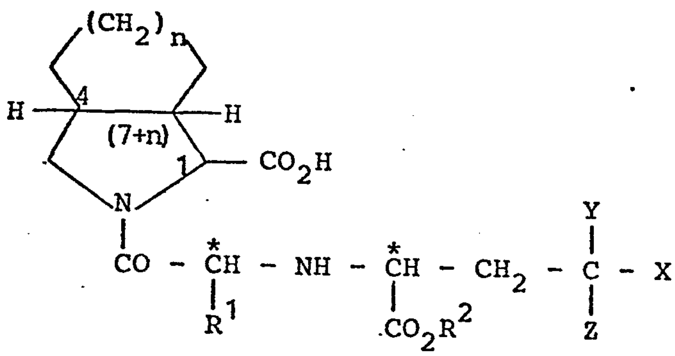

(I)

in der die Wasserstoffatome an den Brückenkopf-C-Atomen 4 und (7+n) zueinander cis- oder trans-konfiguriert sind, wobei die Carboxygruppe am C-Atom 1 zum Wasserstoffatom am C-Atom (7+n) cis- oder transständig orientiert sein kann und worin

n = 1, 2 oder 3,

$R^1$ = Wasserstoff, $(C_1-C_6)$-Alkyl, das gegebenenfalls durch Amino, $(C_1-C_4)$-Acylamino oder Benzoylamino substituiert sein kann, $(C_2-C_6)$-Alkenyl, $(C_5-C_9)$-Cycloalkyl, $C_5-C_9)$-Cycloalkenyl, $(C_5-C_7)$-Cycloalkyl-$(C_1-C_4)$-alkyl, $(C_6-C_{10})$-Aryl oder teilhydriertes $(C_6-C_{10})$-Aryl, das jeweils durch $(C_1-C_4)$-Alkyl, $(C_1-C_2)$-Alkoxy oder Halogen substituiert sein kann, $(C_6-C_{10})$-Aryl-$(C_1-C_4)$alkyl, das wie vorstehend definiert im Aryl-Rest substituiert sein kann, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden Aminosäure,

$R^2$ = Wasserstoff, $(C_1-C_6)$-Alkyl, $(C_2-C_6)$-Alkenyl oder $(C_6-C_{10})$-Aryl-$(C_1-C_4)$-alkyl,

Y = Wasserstoff oder Hydroxy,

Z = Wasserstoff oder

Y und Z = zusammen Sauerstoff und

X = $(C_1-C_6)$-Alkyl, $(C_2-C_6)$-Alkenyl, $(C_5-C_9)$-Cycloalkyl, $(C_6-C_{10})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkyl-amino oder Methylendioxy mono-, di- oder trisubstituiert sein kann, oder Indol-3-yl bedeuten,

sowie deren physiologisch unbedenkliche Salze, dadurch gekennzeichnet

a) daß man eine Verbindung der Formel II

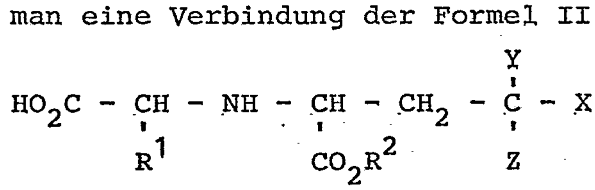

(II)

worin $R^1$, $R^2$, X, Y und Z die Bedeutungen wir in Formel I haben, mit einer Verbindung der Formel III,

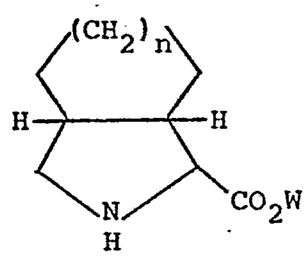

(III)

in der die Konfiguration des bicyclischen Ringsystems und des Substituenten am C-Atom 1 die gleiche ist wie in Formel I und worin

n = 1, 2 oder 3 und

W = einen hydrogenolytisch oder sauer abspaltbaren Rest bedeuten, umsetzt und anschließend den Rest W und gegebenenfalls den Rest $R^2$ unter Bildung der freien Carboxygruppen abspaltet,

b) daß man zur Herstellung der Verbindungen der Formel I, in der Y und Z zusammen Sauerstoff bedeuten,

$b_1$) eine Verbindung der Formel IV,

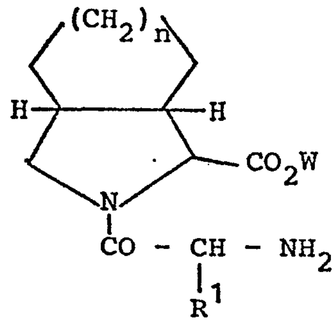

(IV)

in der die Konfiguration des bicyclischen Ringsystems und des Substituenten am C-Atom 1 die gleiche ist und worin n und $R^1$ die Bedeutungen wie in Formel I und W die Bedeutung wie in Formel III besitzen, mit einer Verbindung der Formel V,

$$R^2O_2C - CH = CH - CO - X \qquad (V)$$

worin $R^2$ und X die Bedeutungen wie in Formel I haben, umsetzt und anschließend den Rest W und gegebenenfalls auch den Rest $R^2$ unter Bildung der freien Carboxygruppen abspaltet, oder

$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$$OHC-CO_2R^2 \qquad X-CO-CH_3$$

(VI) (VII)

worin X die Bedeutung wie in Formel I hat, umsetzt und anschließend den Rest W und gegebenenfalls den Rest $R^2$ unter Bildung der freien Carboxygruppen abspaltet, oder

c) daß man zur Herstellung von Verbindungen der Formel I, in der Y und Z jeweils Wasserstoff bedeuten,

$c_1$) eine Verbindung der unter $b_1$) genannten Formel IV mit einer Verbindung der Formel VIII,

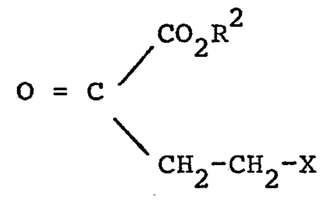

(VIII)

worin $R^2$ und X die Bedeutungen wie in Formel I besitzen, umsetzt, die erhaltenen Schiff-Basen reduziert und anschließend den Rest W und gegebenenfalls den Rest $R^2$ unter Bildung der freien Carboxygruppen abspaltet, oder

$c_2$) eine Verbindung der Formel I, in der Y und Z zusammen Sauerstoff bedeuten, reduziert, oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, reduziert, und die nach a) - d) erhaltenen Verbindungen der Formel I gegebenenfalls in ihre physiologisch unbedenklichen Salze überführt.

2. Verfahren gemäß Anspruch 1, dadurch gekennzeichnet, daß Verbindungen der Formel I hergestellt werden, in denen das C-Atom in Position 1 des bicyclischen Ringsystems sowie die mit einem Stern markierten C-Atome der Seitenkette S-Konfiguration aufweisen.

3. Verfahren gemäß einem der Ansprüche 1 oder 2, dadurch gekennzeichnet, daß in den Verbindungen der Formel I

$R^1$ = Wasserstoff, $(C_1-C_3)$-Alkyl, $(C_2-C_3)$-Alkenyl, Benzyl, 4-Amino-butyl, 4-Methoxybenzyl oder 4-Ethoxybenzyl,

$R^2$ = Wasserstoff, $(C_1-C_4)$-Alkyl oder Benzyl,

X = Phenyl, das durch $(C_1-C_2)$-Alkyl, $(C_1-C_2)$-Alkoxy, Hydroxy, Fluor, Chlor, Brom, Amino, $(C_1-C_4)$-Alkylamino,

Di-($C_1$-$C_4$)alkyl-amino, Nitro oder Methylendioxy mono- oder disubstituiert oder im Falle von Methoxy, trisubstituiert sein kann, bedeuten.

4. Verfahren gemäß einem der Ansprüche 1 bis 3, dadurch gekennzeichnet, daß in den Verbindungen der Formel I n = 1 oder 2, $R^1$ = Methyl, 4-Methoxybenzyl oder 4-Ethoxybenzyl und X = Phenyl bedeuten.

5. Verfahren gemäß einem der Ansprüche 1 bis 4, dadurch gekennzeichnet, daß in den Verbindungen der Formel I $R^2$ = Wasserstoff oder Ethyl bedeutet.

6. Verfahren zur Herstellung einer Verbindung der Formel I gemäß einem der Ansprüche 1 bis 5 zur Verwendung als Heilmittel.

7. Verfahren zur Herstellung eines Mittels, enthaltend eine Verbindung der Formel I, dadurch gekennzeichnet, daß man die Verbindung der Formel I in eine geeignete Zubereitungsform bringt.

8. Verfahren zur Herstellung von Verbindungen der Formel III',

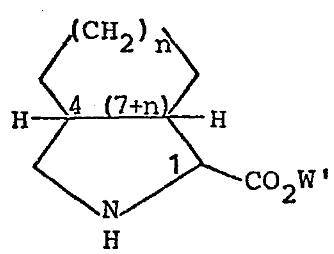

(III')

in der die C-Atome 1,4 und (7+n) dieselbe Konfiguration wie in Formel III besitzen und worin

n = 1, 2 oder 3

W'= Wasserstoff oder ein hydrogenolytisch oder sauer abspaltbaren Rest bedeuten,

a) daß man eine Verbindung der Formel IX,

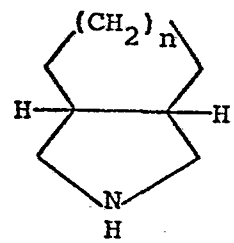

(IX)

worin die H-Atome an den C-Atomen 4 und (7+n) zueinander cis- oder trans-konfiguriert sind und worin n die Zahl 1, 2 oder 3 bedeutet, acyliert, anschließend mit einem Alkohol in Gegenwart eines Leitsalzes zu einer Verbindung der Formel X,

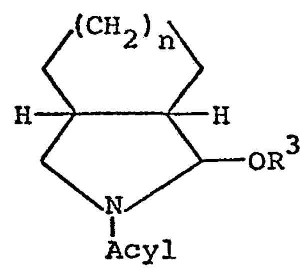

(X)

worin n die oben genannte Bedeutung hat und $R^3$ = $(C_1-C_4)$-Alkyl bedeutet, anodisch oxydiert, diese mit Trimethylsilylcyanid in Gegenwart einer Lewis-Säure zu einer Verbindung der Formel XI,

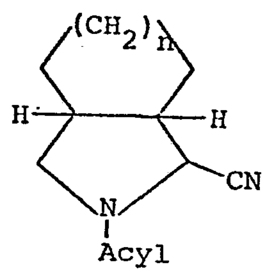

(XI)

worin die H-Atome an den C-Atomen 4 und (7+n) zueinander cis- oder trans-konfiguriert sind und wobei die CN-Gruppe sich in cis-Stellung zum H-Atom am C-Atom (7+n) befindet und worin n die oben genannte Bedeutung hat, umsetzt und diese durch Einwirkung von Säuren

oder Basen zu einer Verbindung der Formel III' mit W' = Wasserstoff hydrolysiert und letztere gegebenenfalls verestert, oder

b) daß man eine Verbindung der Formel XII,

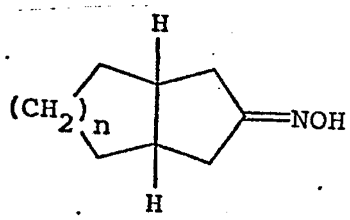

(XII)

worin die H-Atome an den C-Atomen 4 und (7+n) cis- oder trans-konfiguriert sind und n die oben genannte Bedeutung besitzt in einer Beckmann-Umlagerung zu einer Verbindung der Formel XIII

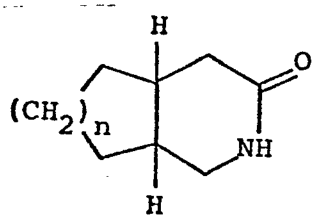

(XIII)

worin n die obengenannte Bedeutung besitzt, umsetzt, diese zu einer Verbindung der Formel XIV,

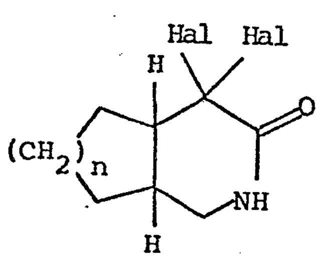

(XIV)

worin n die obengenannte Bedeutung besitzt und Hal ein Halogenatom bedeutet, halogeniert, diese zu einer Verbindung der Formel XV,